# EUROPEAN PATENT SPECIFICATION

(11) **EP 3 399 933 B1**
(45) Date of publication and mention of the grant of the patent: **01.09.2021**
(21) Application number: 17736218.3
(22) Date of filing: 04.01.2017
(51) Int. Cl.: A61B 18/14, A61N 1/05, A61N 1/32, A61N 1/362, A61B 18/00, A61B 18/12, A61B 90/00

(54) **SYSTEMS FOR DELIVERY OF PULSED ELECTRIC FIELD ABLATIVE ENERGY TO ENDOCARDIAL TISSUE**
SYSTEME ZUR ABGABE VON GEPULSTER ELEKTRISCHER FELDABLATIONSENERGIE AN ENDOKARDIALES GEWEBE
SYSTÈMES D'APPLICATION D'UNE ÉNERGIE D'ABLATION À CHAMP ÉLECTRIQUE PULSÉ SUR UN TISSU DE L'ENDOCARDE

(30) Priority: 05.01.2016 US 201662274943 P
(43) Date of publication of application: 14.11.2018
(73) Proprietor: Farapulse, Inc., Menlo Park CA 94025 (US)
(72) Inventor: PAGEARD, Jean-Luc, Montreal Québec H3J 2W3 (CA); VISWANATHAN, Raju, Mountain View California 94041 (US); LONG, Gary L., Cincinnati Ohio 45227 (US)
(74) Representative: Cooley (UK) LLP
(86) International application number: PCT/US2017/012099
(87) International publication number: WO 2017/120169

(56) References cited:
- WO-A1-94/07413
- WO-A1-2015/192018
- WO-A2-2015/171921
- WO-A2-2015/171921
- US-A- 5 702 438
- US-A- 6 071 281
- US-A1- 2002 183 638
- US-A1- 2005 222 632
- US-A1- 2010 312 300
- US-A1- 2012 197 100
- US-A1- 2014 058 377
- US-A1- 2014 142 408
- US-A1- 2014 194 716
- US-A1- 2014 235 988
- US-A1- 2015 320 481
- US-A1- 2015 342 532
- US-A1- 2015 351 836
- US-A1- 2015 351 836
- US-B2- 7 387 630

## Description

### BACKGROUND

The generation of pulsed electric fields for tissue therapeutics has moved from the laboratory to clinical applications over the past two decades, while the effects of brief pulses of high voltages and large electric fields on tissue have been investigated for the past forty years or more. Application of brief high DC voltages to tissue may generate locally high electric fields typically in the range of hundreds of volts per centimeter that disrupt cell membranes by generating pores in the cell membrane. While the precise mechanism of this electrically-driven pore generation or electroporation continues to be studied, it is thought that the application of relatively brief and large electric fields generates instabilities in the lipid bilayers in cell membranes, causing the occurrence of a distribution of local gaps or pores in the cell membrane. This electroporation may be irreversible if the applied electric field at the membrane is larger than a threshold value such that the pores do not close and remain open, thereby permitting exchange of biomolecular material across the membrane leading to necrosis and/or apoptosis (cell death). Subsequently, the surrounding tissue may heal naturally.

While pulsed DC voltages may drive electroporation under the right circumstances, there remains an unmet need for thin, flexible, atraumatic devices that effectively deliver high DC voltage electroporation ablation therapy selectively to endocardial tissue in regions of interest while minimizing damage to healthy tissue.

WO 94/07413 discloses catheters and methods for performing cardiac diagnosis and treatment. US 2014/235988 discloses methods for simultaneous cardiac substrate mapping using spatial correlation maps between neighboring unipolar electrograms.

US 2014/058377 discloses methods and apparatus for renal neuromodulation.

US 2015/351836 discloses a unipolar and/or bipolar ablation catheter.

WO 2015/171921 discloses methods and apparatus for selective tissue ablation.

### SUMMARY

In accordance with the invention, there is provided an apparatus claim as defined in claim 1. Optional features are defined in the dependent claims.

Described here are systems, devices, and methods for ablating tissue through irreversible electroporation. The methods, however, do not form part of the invention. Generally, a system for delivering a pulse waveform to tissue includes a signal generator configured for generating a pulse waveform and an ablation device coupled to the signal generator and configured to receive the pulse waveform. The ablation device includes a set of spines. The ablation device is configured for delivering the pulse waveform to tissue during use via one or more spine of the set of spines. Each spine includes a set of independently addressable electrodes formed on a surface of the spine. Each electrode of the set of electrodes has an insulated electrical lead associated therewith. The insulated electrical leads are disposed in a body of the spine.

According to the invention, the insulated electrical leads are configured for sustaining a voltage potential of at least about 700 V without dielectric breakdown of its corresponding insulation. The system may further include a cardiac stimulator configured for generating a pacing signal for cardiac stimulation during use. The cardiac stimulator may be communicably coupled to the signal generator and further configured for transmitting an indication of the pacing signal to the signal generator. The signal generator further may be configured for generating the pulse waveform in synchronization with the indication of the pacing signal. The signal generator may be further configured for generating the pulse waveform with a time offset with respect to the indication of the pacing signal. The ablation device may further include a distal cap configured to tether the set of spines to form a distal end of the ablation device. The ablation device may further include a spine wire configured for electrically coupling the electrodes between a pair of spines of the set of spines. The pair of spines may be adjacent to each other. The spine wire may be a first spine wire of a set of spine wires. Each spine wire of the set of spine wires may couple electrodes between a different pair of spines of the set of spines. The set of spine wires may form a continuous loop between the electrodes coupled thereto. The set of spine wires may be a first set of spine wires coupling the electrodes across the set of spines. The ablation device may further include a second set of spine wires coupling the electrodes across the set of spines. The second set of spine wires may couple different electrodes across the set of spines than the first set of spine wires. The first set of spine wires may form a first continuous loop between the electrodes coupled thereto and the second set of spine wires may form a second continuous loop between the electrodes coupled thereto. The first continuous loop may be electrically isolated from the second continuous loop. The first spine wire of the set of spine wires may couple electrodes between a first spine and a second spine of the set of spines. A second spine wire of the set of spine wires may couple electrodes between the first spine and a third spine of the set of spines. The first spine wire of the set of spine wires may couple the electrodes between a first spine and a second spine of the set of spines. A second spine wire of the set of spine wires may couple the electrodes between a third spine and a fourth spine of the set of spines.

In some embodiments, the electrodes can be electrically connected together (so that they are at the same electric potential) at a proximal portion of the shaft of the device, or within a device handle. In these embodiments, the electrical connections between electrodes can be similar to the variants described herein. For example, a loop of electrodes at the same electric potential can be formed, or adjacent electrodes on adjacent spines can be at the same electric potential, or in general a first set of electrodes on the spines can be maintained at the same electric potential as a second set of electrodes on the spines by suitable electrical connections between electrode leads at a proximal portion of the shaft of the device, or within a device handle.

In some embodiments, the set of spines may include from 3 spines to 20 spines. The set of electrodes may include from 2 electrodes to 64 electrodes. Each electrode of the set of electrodes may have a surface area from about 0.5 mm² to about 20 mm². Each spine of the set of spines may have a cross-sectional area from about 0.2 mm² to about 15 mm². The set of spines may form a delivery assembly at a distal portion of the ablation device. The delivery assembly may be configured for transforming between a first configuration where the set of spines bow radially outward from a longitudinal axis of the ablation device, and a second configuration where the set of spines are arranged generally substantially parallel to the longitudinal axis of the ablation device. The delivery assembly may have a maximum cross-sectional area from about 1 mm² to about 25 mm². The system may further include a handle coupled to the set of spines. The handle may be configured for affecting transformation of the set of spines between the first configuration and the second configuration. The set of spines may include a first spine and a second spine adjacent to the first spine. An electrode of the first spine may be disposed closer to a distal end of the ablation device relative to an electrode of the second spine. The set of spines may be present in a distal portion of the ablation device. The electrical leads of at least two electrodes of the set of electrodes may be electrically coupled at or near a proximal portion of the ablation device.

In embodiments, the set of spines forms an expanded structure during use. A cross-sectional area at a first plane of the expanded structure may be different than a cross-sectional area at a second plane of the expanded structure. At least one electrode of the set of electrodes, or at least a portion thereof, may be disposed in a distal end plane of the expanded structure. The set of spines may converge at their distal ends at a point inside the expanded structure.

In some embodiments, the pulse waveform may include a first level of a hierarchy of the pulse waveform including a pulse. Each pulse may have a pulse time duration and a first time interval separating successive pulses. A second level of the hierarchy of the pulse waveform includes a plurality of pulses as a second set of pulses and a second time interval separating successive second sets of pulses. The second time interval may be at least three times the duration of the first time interval. A third level of the hierarchy of the pulse waveform includes a plurality of second sets of pulses as a third set of pulses and a third time interval separating successive third sets of pulses. The third time interval may be at least thirty times the duration of the second level time interval.

Also described here are methods for treating atrial fibrillation via irreversible electroporation. These methods, however, do not form part of the claimed invention. In general, these methods include the steps of generating a pulse waveform and delivering the pulse waveform to a pulmonary vein ostium of a heart of a patient via one or more spines of a set of spines of an ablation device. Each spine may include a set of independently addressable electrodes formed on a surface of the spine. Each electrode of the set of electrodes may have an insulated electrical lead associated therewith. The insulated electrical leads may be disposed in a body of the spine.

In some embodiments, the ablation device may be disposed in the endocardial space of the left atrium of the heart, and in contact with the pulmonary vein ostium. Each insulated electrical lead may be configured for sustaining a voltage potential of at least about 700 V without dielectric breakdown of its corresponding insulation. A pacing signal may be generated for cardiac stimulation of the heart. The pacing signal may be applied to the heart, and the pulse waveform generated in synchronization with the pacing signal. The pulse waveform may include a time offset with respect to the pacing signal, and such an offset is understood to be encompassed by the term "synchronization" as used herein. A group of electrodes on the set of spines may include the set of electrodes of each spine of the set of spines. The method may further include the steps of configuring a first electrode of the group of electrodes as an anode, configuring a second electrode of the group of electrodes as a cathode, and delivering the pulse waveform to the first electrode and the second electrode.

In some examples, the method may further include the steps of configuring a first set of electrodes of a first spine of the set of spines as anodes, configuring a second set of electrodes of a second spine of the set of spines as cathodes, and delivering the pulse waveform to the first set of electrodes and the second set of electrodes. The ablation device may further include a first set of spine wires and a second set of spine wires. Each spine wire may be configured for electrically coupling the electrodes between a pair of spines of the set of spines. The second set of spine wires may couple different electrodes across the set of spines than the first set of spine wires. The first set of spine wires may form a first continuous loop between the electrodes coupled thereto and the second set of spine wires forms a second continuous loop between the electrodes coupled thereto. The first continuous loop may be electrically isolated from the second continuous loop. The method may further include the steps of configuring the electrodes coupled to the first continuous loop as anodes, configuring the electrodes coupled to the second continuous loop as cathodes, and delivering the pulse waveform to the electrodes coupled to the first continuous loop and to the electrodes coupled to the second continuous loop.

In some embodiments, the spine wires, or the first set of spine wires and the second set of spine wires, may be replaced by electrical connections between the appropriate leads (so that they are at the same electric potential) at a proximal portion of the shaft of the device, or within a device handle. In these embodiments, the electrical connections between electrodes can be similar to the variants described above. For example, a loop of electrodes at the same electric potential can be formed, or adjacent electrodes on adjacent spines can be at the same electric potential, or in general a first set of electrodes on the spines can be maintained at the same electric potential as a second set of electrodes on the spines by suitable electrical connections between electrode leads at a proximal portion of the shaft of the device, or within a device handle.

In some embodiments, the ablation device may further include a first spine wire coupling the electrodes between a first spine and a second spine of the set of spines. A second spine wire may couple the electrodes between the first spine and a third spine of the set of spines. The method may further include the steps of configuring the electrodes coupled by the first spine wire and the second spine wire as an anode or as a cathode, and delivering the pulse waveform to the electrodes coupled by the first spine wire and the second spine wire. The ablation device may further include a first spine wire coupling the electrodes between a first spine and a second spine of the set of spines. A second spine wire may couple the electrodes between a third spine and a fourth spine of the set of spines. The method may further include the steps of configuring the electrodes coupled by the first spine wire as an anode, configuring the electrodes coupled by the second spine wire as a cathode, and delivering the pulse waveform to the anode and cathode. In some embodiments, the spine wires, or the first spine wire and the second spine wire, may be replaced by electrical connections between the appropriate leads (so that they are at the same electric potential) at a proximal portion of the shaft of the device, or within a device handle. In these embodiments, the electrical connections between electrodes can be similar to the variants described above, and the method may further include the steps of configuring a first set of electrically coupled electrodes as an anode, configuring a second set of electrically coupled electrodes as a cathode, and delivering the pulse waveform to the anode and cathode.

The set of spines may form a delivery assembly at a distal portion of the ablation device. The delivery assembly may be configured for transforming between a first configuration where the set of spines bow radially outward from a longitudinal axis of the ablation device. The method may further include the steps of disposing the delivery assembly in the first configuration in contact with the pulmonary vein ostium, and transforming the delivery assembly to the second configuration prior to the delivering the pulse waveform. The set of spines may be present in a distal portion of the ablation device. The electrical leads of at least two electrodes of the set of electrodes may be electrically coupled at or near a proximal portion of the ablation device.

In some embodiments, the pulse waveform may include a first level of a hierarchy of the pulse waveform including a first set of pulses. Each pulse may have a pulse time duration and a first time interval separating successive pulses. A second level of the hierarchy of the pulse waveform may include a plurality of first sets of pulses as a second set of pulses. A second time interval may separate successive first sets of pulses. The second time interval may be at least three times the duration of the first time interval. A third level of the hierarchy of the pulse waveform may include a plurality of second sets of pulses as a third set of pulses. A third time interval may separate successive second sets of pulses. The third time interval may be at least thirty times the duration of the second level time interval.

In some embodiments, a system for ablating tissue described here may include a signal generator configured for generating a pulse waveform. The system also includes an ablation device that may be coupled to the signal generator and configured to deliver the pulse waveform to tissue. The ablation device may include a set of spines, and the ablation device may be configured for delivering the pulse waveform to tissue during use via one or more spines of the set of spines. Each spine may include a set of independently addressable electrodes formed on a surface of the spine. Each electrode of the set of electrodes may have an insulated electrical lead associated therewith. The insulated electrical leads may be disposed in a body of the spine.

In some embodiments, a system may include a signal generator configured for generating a pulse waveform and an ablation device coupled to the signal generator. The ablation device may include a set of electrodes and be configured to receive the pulse waveform. The ablation device may be configured for delivering the pulse waveform to tissue during use via the set of electrodes. Each electrode of the set of electrodes may have an insulated electrical lead associated therewith. Each insulated electrical lead may be configured for sustaining a voltage potential of at least about 700 V without dielectric breakdown of its corresponding insulation. In some embodiments the system may further include a cardiac stimulator configured for generating a pacing signal for cardiac stimulation during use. The cardiac stimulator may be communicably coupled to the signal generator and further configured for transmitting an indication of the pacing signal to the signal generator. The signal generator may be further configured for generating the pulse waveform in synchronization with the indication of the pacing signal.

In some embodiments, the signal generator may be further configured for generating the pulse waveform with a time offset with respect to the indication of the pacing signal. In other embodiments, the system may further include a guidewire having a nonlinear distal portion. The ablation device may be configured for being disposed over the guidewire during use. In some other embodiments, the ablation device may include a nonlinear distal portion, the nonlinear distal portion having one or more electrodes of the set of electrodes disposed thereon. The pulse waveform may include a first level of a hierarchy of the pulse waveform including a pulse. Each pulse may have a pulse time duration and a first time interval separating successive pulses. A second level of the hierarchy of the pulse waveform may include a plurality of pulses as a second set of pulses. A second time interval may separate successive second sets of pulses. The second time interval may be at least three times the duration of the first time interval. A third level of the hierarchy of the pulse waveform may include a plurality of second sets of pulses as a third set of pulses. A third time interval may separate successive third sets of pulses. The third time interval may be at least thirty times the duration of the second level time interval.

### BRIEF DESCRIPTION OF THE DRAWINGS

FIG. 1 is a block diagram of an electroporation system, according to embodiments.
FIG. 2 is a perspective view of an ablation catheter, not forming part of the invention.
FIG. 3 is a perspective view of an ablation catheter, not forming part of the invention.
FIG. 4 is a perspective view of an ablation catheter, not forming part of the invention.
FIG. 5 is a detailed perspective view of a distal portion of an ablation catheter, not forming part of the invention.
FIG. 6 is a side view of an ablation catheter, not forming part of the invention.
FIG. 7 is a side view of an ablation catheter, not forming part of the invention.
FIGS. 8A-8B are side and front cross-sectional views, respectively, of an ablation catheter, not forming part of the invention.
FIGS. 9A-9E are side views of an ablation catheter, according to other embodiments.
FIG. 10 is a perspective view of a balloon ablation catheter disposed in a left atrial chamber of a heart, according to other examples, not forming part of the invention.
FIG. 11 is a cross-sectional view of a balloon ablation catheter disposed in a left atrial chamber of a heart, according to other examples, not forming part of the invention.
FIGS. 12A-12B are schematic views of a return electrode of an ablation system, according to embodiments.
FIG. 13 illustrates a method for tissue ablation, not forming part of the invention.
FIG. 14 illustrates a method for tissue ablation, not forming part of the invention.
FIG. 15 is an illustration of the ablation catheter depicted in FIG. 2 disposed in a left atrial chamber of a heart.
FIG. 16 is an illustration of the ablation catheter depicted in FIG. 3 disposed in a left atrial chamber of a heart.
FIG. 17 is an illustration of two of the ablation catheters depicted in FIG. 4 disposed in a left atrial chamber of a heart.
FIG. 18 is an illustration of the ablation catheter depicted in FIG. 5 disposed in a left atrial chamber of a heart.
FIGS. 19A-19B are schematic perspective and cross-sectional views, respectively, of a set of electrodes disposed in a pulmonary vein ostium.
FIGS. 20A-20B are schematic perspective and cross-sectional views, respectively, of an electric field generated by electrodes disposed in a pulmonary vein ostium.
FIG. 21 is an example waveform showing a sequence of voltage pulses with a pulse width defined for each pulse.
FIG. 22 schematically illustrates a hierarchy of pulses showing pulse widths, intervals between pulses, and groupings of pulses.
FIG. 23 provides a schematic illustration of a nested hierarchy of monophasic pulses displaying different levels of nested hierarchy.
FIG. 24 is a schematic illustration of a nested hierarchy of biphasic pulses displaying different levels of nested hierarchy.
FIG. 25 illustrates schematically a time sequence of electrocardiograms and cardiac pacing signals together with atrial and ventricular refractory time periods and indicating a time window for irreversible electroporation ablation.

### DETAILED DESCRIPTION

Described herein are systems, devices, and methods for selective and rapid application of pulsed electric fields to ablate tissue by irreversible electroporation. The methods, however, do not form part of the invention. Generally, the systems, devices, and methods described herein may be used to generate large electric field magnitudes at desired regions of interest and reduce peak electric field values elsewhere in order to reduce unnecessary tissue damage and electrical arcing. An irreversible electroporation system as described herein includes a signal generator and a processor configured to apply one or more voltage pulse waveforms to a selected set of electrodes of an ablation device to deliver energy to a region of interest (e.g., ablation energy for a set of tissue in a pulmonary vein ostium). The pulse waveforms disclosed herein may aid in therapeutic treatment of a variety of cardiac arrhythmias (e.g., atrial fibrillation). In order to deliver the pulse waveforms generated by the signal generator, each electrodes of the ablation device has an insulated electrical lead configured for sustaining a voltage potential of at least about 700 V without dielectric breakdown of its corresponding insulation. The electrodes are independently addressable such that each electrode may be controlled (e.g., deliver energy) independently of any other electrode of the device. In this manner, the electrodes may deliver different energy waveforms with different timing synergistically for electroporation of tissue.

The term "electroporation" as used herein refers to the application of an electric field to a cell membrane to change the permeability of the cell membrane to the extracellular environment. The term "reversible electroporation" as used herein refers to the application of an electric field to a cell membrane to temporarily change the permeability of the cell membrane to the extracellular environment. For example, a cell undergoing reversible electroporation can observe the temporary and/or intermittent formation of one or more pores in its cell membrane that close up upon removal of the electric field. The term "irreversible electroporation" as used herein refers to the application of an electric field to a cell membrane to permanently change the permeability of the cell membrane to the extracellular environment. For example, a cell undergoing irreversible electroporation can observe the formation of one or more pores in its cell membrane that persist upon removal of the electric field.

Pulse waveforms for electroporation energy delivery as disclosed herein may enhance the safety, efficiency and effectiveness of energy delivery to tissue by reducing the electric field threshold associated with irreversible electroporation, thus yielding more effective ablative lesions with a reduction in total energy delivered. In some embodiments, the voltage pulse waveforms disclosed herein may be hierarchical and have a nested structure. For example, the pulse waveform may include hierarchical groupings of pulses having associated timescales. In some embodiments, the methods, systems, and devices disclosed herein may comprise one or more of the methods, systems, and devices described in International Application Serial No. PCT/US2016/057664, filed on October 19, 2016, and titled "SYSTEMS, APPARATUSES AND METHODS FOR DELIVERY OF ABLATIVE ENERGY TO TISSUE".

In some embodiments, the systems may further include a cardiac stimulator used to synchronize the generation of the pulse waveform to a paced heartbeat. The cardiac stimulator may electrically pace the heart with a cardiac stimulator and ensure pacing capture to establish periodicity and predictability of the cardiac cycle. A time window within a refractory period of the periodic cardiac cycle may be selected for voltage pulse waveform delivery. Thus, voltage pulse waveforms may be delivered in the refractory period of the cardiac cycle so as to avoid disruption of the sinus rhythm of the heart. In some embodiments, an ablation device may include one or more catheters, guidewires, balloons, and electrodes. The ablation device may transform into different configurations (e.g., compact and expanded) to position the device within an endocardial space. In some embodiments, the system may optionally include one or more return electrodes.

Generally, to ablate tissue, one or more catheters may be advanced in a minimally invasive fashion through vasculature to a target location. In a cardiac application, the electrodes through which the voltage pulse waveform is delivered may be disposed on an epicardial device or on an endocardial device. The methods described here may include introducing a device into an endocardial space of the left atrium of the heart and disposing the device in contact with a pulmonary vein ostium. A pulse waveform may be generated and delivered to one or more electrodes of the device to ablate tissue. In some embodiments, the pulse waveform may be generated in synchronization with a pacing signal of the heart to avoid disruption of the sinus rhythm of the heart. In some embodiments, the electrodes may be configured in anode-cathode subsets. The pulse waveform may include hierarchical waveforms to aid in tissue ablation and reduce damage to healthy tissue.

### I. Systems

### Overview

Disclosed herein are systems and devices configured for tissue ablation via the selective and rapid application of voltage pulse waveforms to aid tissue ablation, resulting in irreversible electroporation. Generally, a system for ablating tissue described here includes a signal generator and an ablation device having one or more electrodes for the selective and rapid application of DC voltage to drive electroporation. As described herein, the systems and devices may be deployed epicardially and/or endocardially to treat atrial fibrillation. Voltages may be applied to a selected subset of the electrodes, with independent subset selections for anode and cathode electrode selections. A pacing signal for cardiac stimulation may be generated and used to generate the pulse waveform by the signal generator in synchronization with the pacing signal.

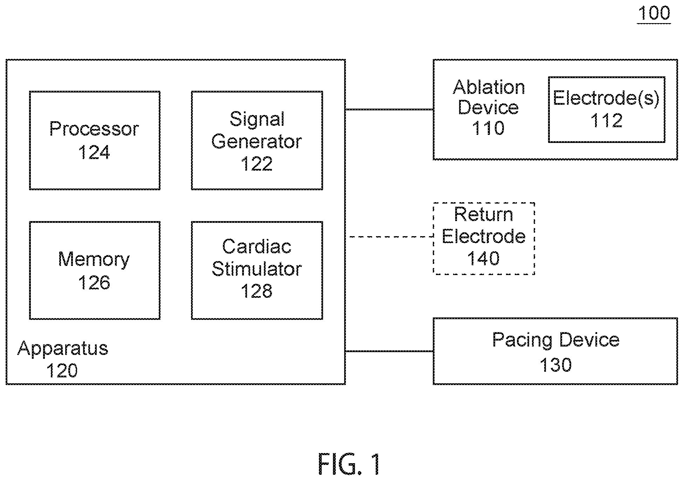

Generally, the systems and devices described herein include one or more catheters configured to ablate tissue in a left atrial chamber of a heart. FIG. 1 illustrates an ablation system (100) configured to deliver voltage pulse waveforms. The system (100) may include an apparatus (120) including a signal generator (122), processor (124), memory (126), and cardiac stimulator (128). The apparatus (120) may be coupled to an ablation device (110), and optionally to a pacing device (130) and/or an optional return electrode (140) (e.g., a return pad, illustrated here with dotted lines).

The signal generator (122) may be configured to generate pulse waveforms for irreversible electroporation of tissue, such as, for example, pulmonary vein ostia. For example, the signal generator (122) may be a voltage pulse waveform generator and deliver a pulse waveform to the ablation device (110). The return electrode (140) may be coupled to a patient (e.g., disposed on a patient's back) to allow current to pass from the ablation device (110) through the patient and then to the return electrode (140) to provide a safe current return path from the patient (not shown). The processor (124) may incorporate data received from memory (126), cardiac stimulator (128), and pacing device (130) to determine the parameters (e.g., amplitude, width, duty cycle, etc.) of the pulse waveform to be generated by the signal generator (122). The memory (126) may further store instructions to cause the signal generator (122) to execute modules, processes and/or functions associated with the system (100), such as pulse waveform generation and/or cardiac pacing synchronization. For example, the memory (126) may be configured to store pulse waveform and/or heart pacing data for pulse waveform generation and/or cardiac pacing, respectively.

In some embodiments, the ablation device (110) includes a catheter configured to receive and/or deliver the pulse waveforms described in more detail below. For example, the ablation device (110) may be introduced into an endocardial space of the left atrium and positioned to align one or more electrodes (112) to one or more pulmonary vein ostia, and then deliver the pulse waveforms to ablate tissue. The ablation device (110) includes a set of independently addressable electrodes. Each electrode includes an insulated electrical lead configured to sustain a voltage potential of at least about 700 V without dielectric breakdown of its corresponding insulation. In some embodiments, the insulation on each of the electrical leads may sustain an electrical potential difference of up to about 1,500 V across its thickness without dielectric breakdown. For example, the electrodes (112) may be grouped into one or more anode-cathode subsets such as, for example, a subset including one anode and one cathode, a subset including two anodes and two cathodes, a subset including two anodes and one cathode, a subset including one anode and two cathodes, a subset including three anodes and one cathode, a subset including three anodes and two cathodes, and/or the like.

The pacing device (130) may be suitably coupled to the patient (not shown) and configured to receive a heart pacing signal generated by the cardiac stimulator (128) of the apparatus (120) for cardiac stimulation. An indication of the pacing signal may be transmitted by the cardiac stimulator (128) to the signal generator (122). Based on the pacing signal, an indication of a voltage pulse waveform may be selected, computed, and/or otherwise identified by the processor (124) and generated by the signal generator (122). In some embodiments, the signal generator (122) is configured to generate the pulse waveform in synchronization with the indication of the pacing signal (e.g., within a common refractory window). For example, in some embodiments, the common refractory window may start substantially immediately following a ventricular pacing signal (or after a very small delay) and last for a duration of approximately 130 ms or less thereafter. In such embodiments, an entire pulse waveform may be delivered within this duration.

The processor (124) may be any suitable processing device configured to run and/or execute a set of instructions or code. The processor may be, for example, a general purpose processor, a Field Programmable Gate Array (FPGA), an Application Specific Integrated Circuit (ASIC), a Digital Signal Processor (DSP), and/or the like. The processor may be configured to run and/or execute application processes and/or other modules, processes and/or functions associated with the system and/or a network associated therewith (not shown). The underlying device technologies may be provided in a variety of component types, e.g., metal-oxide semiconductor field-effect transistor (MOSFET) technologies like complementary metal-oxide semiconductor (CMOS), bipolar technologies like emitter-coupled logic (ECL), polymer technologies (e.g., silicon-conjugated polymer and metal-conjugated polymer-metal structures), mixed analog and digital, and/or the like.

The memory (126) may include a database (not shown) and may be, for example, a random access memory (RAM), a memory buffer, a hard drive, an erasable programmable read-only memory (EPROM), an electrically erasable read-only memory (EEPROM), a read-only memory (ROM), Flash memory, etc. The memory (126) may store instructions to cause the processor (124) to execute modules, processes and/or functions associated with the system (100), such as pulse waveform generation and/or cardiac pacing.

The system (100) may be in communication with other devices (not shown) via, for example, one or more networks, each of which may be any type of network. A wireless network may refer to any type of digital network that is not connected by cables of any kind. However, a wireless network may connect to a wireline network in order to interface with the Internet, other carrier voice and data networks, business networks, and personal networks. A wireline network is typically carried over copper twisted pair, coaxial cable or fiber optic cables. There are many different types of wireline networks including, wide area networks (WAN), metropolitan area networks (MAN), local area networks (LAN), campus area networks (CAN), global area networks (GAN), like the Internet, and virtual private networks (VPN). Hereinafter, network refers to any combination of combined wireless, wireline, public and private data networks that are typically interconnected through the Internet, to provide a unified networking and information access solution.

### Ablation Device

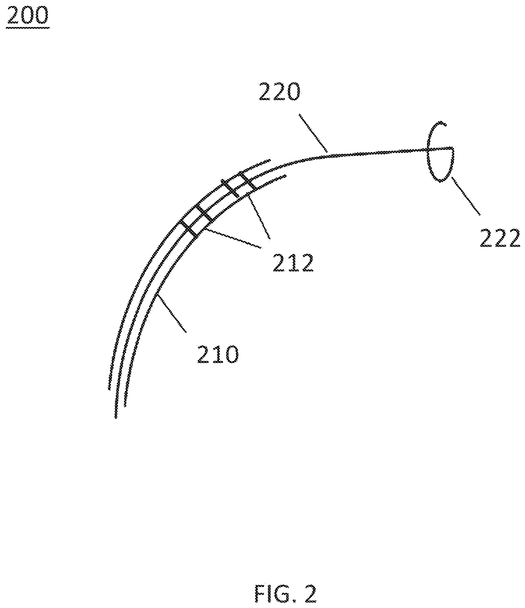

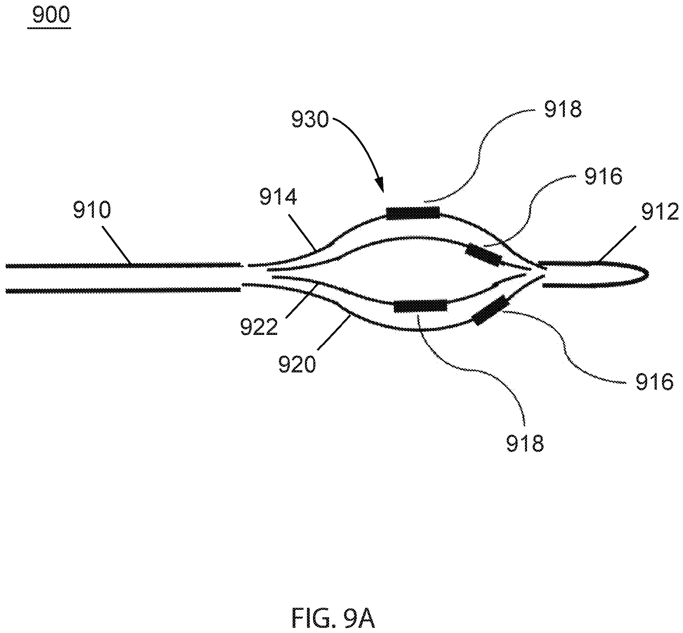

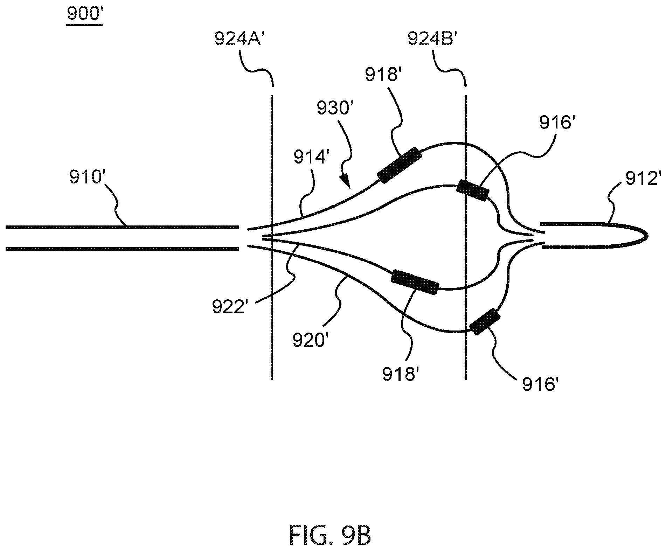

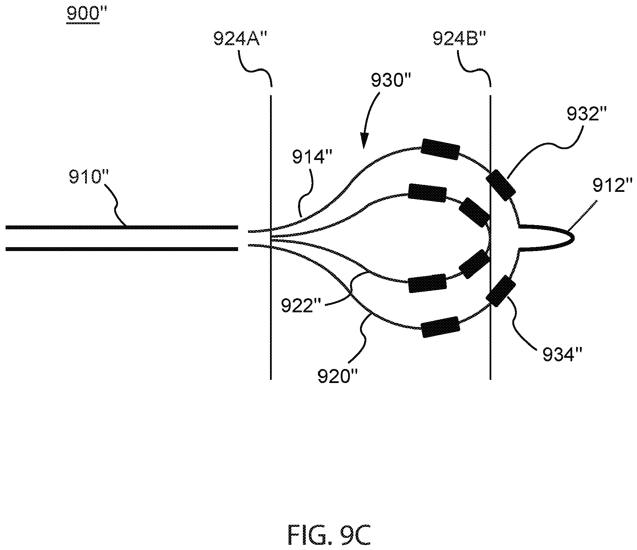

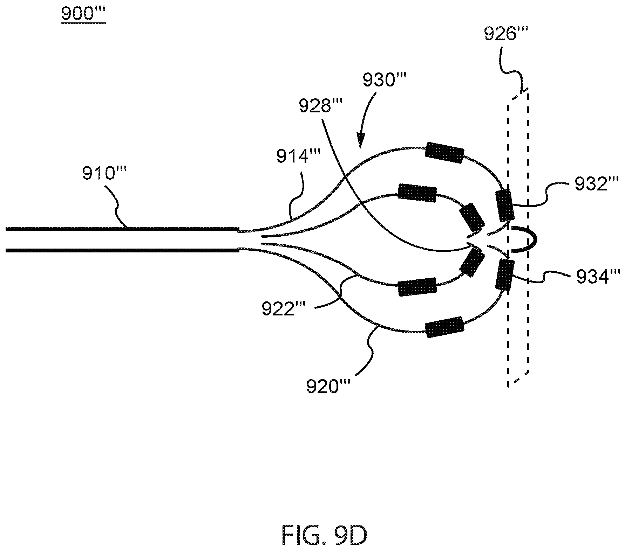

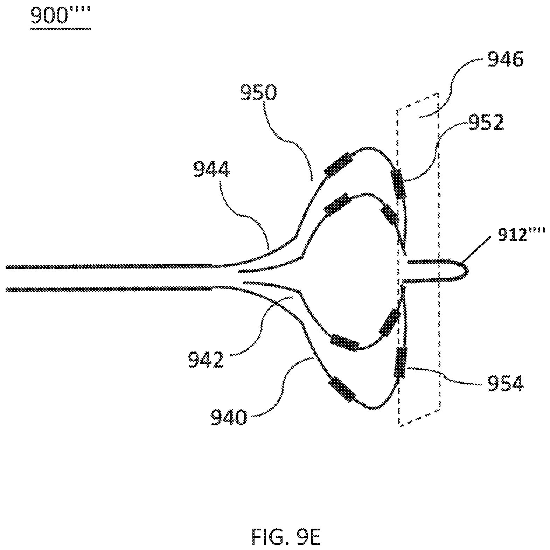

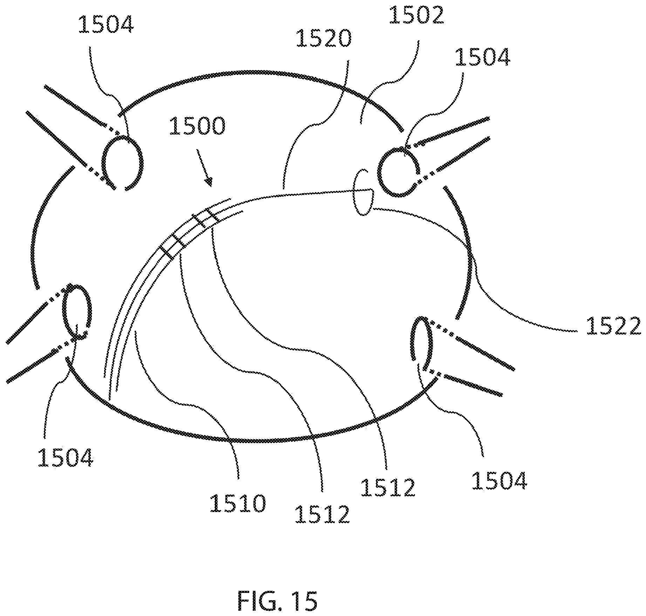

The systems described include may include one or more multi-electrode ablation devices configured to ablate tissue in a left atrial chamber of a heart for treating atrial fibrillation. It should be noted that the ablation devices described here below with reference to FIG. 2-8, 10-11 and 15-20 do not form part of the invention. Only the devices described with reference to FIG. 9 form part of the invention and only provided that the set of spines is deployable to form an asymmetric expanded structure in which a subset of the set of electrodes lies in a distal end plane that is orthogonal to a longitudinal axis of the catheter shaft and passes through a distal boundary of the expanded structure (as illustrated by FIG. 9D). FIG. 2 is a perspective view of an ablation device (200) (e.g., structurally and/or functionally similar to the ablation device (110)) including a catheter (210) and a guidewire (220) slidable within a lumen of the catheter (210). The guidewire (220) may include a nonlinear distal portion (222) and the catheter (210) may be configured to be disposed over the guidewire (220) during use. The distal portion (222) of the guidewire (220) may be shaped to aid placement of the catheter (210) in a lumen of the patient. For example, a shape of the distal portion (222) of the guidewire (220) may be configured for placement in a pulmonary vein ostium and/or the vicinity thereof, as described in more detail with respect to FIG. 15. The distal portion (222) of the guidewire (220) may include and/or be formed in an atraumatic shape that reduces trauma to tissue (e.g., prevents and/or reduces the possibility of tissue puncture). For example, the distal portion (222) of the guidewire (220) may include a nonlinear shape such as a circle, loop (as illustrated in FIG. 2), ellipsoid, or any other geometric shape. In some embodiments, the guidewire (220) may be configured to be resilient such that the guidewire having a nonlinear shape may conform to a lumen of the catheter (210) when disposed in the catheter (210), and re-form/otherwise regain the nonlinear shape when advanced out of the catheter (210). In other embodiments, the catheter (210) may similarly be configured to be resilient such as for aiding advancement of the catheter (210) through a sheath (not shown). The shaped distal portion (222) of the guidewire (220) may be angled relative to the other portions of the guidewire (220) and catheter (210). The catheter (210) and guidewire (220) may be sized for advancement into an endocardial space (e.g., left atrium). A diameter of the shaped distal portion (222) of the guidewire (220) may be about the same as a diameter of a lumen in which the catheter (230) is to be disposed.

The catheter (210) may be slidably advanced over the guidewire (220) so as to be disposed over the guidewire (220) during use. The distal portion (222) of the guidewire (220) disposed in a lumen (e.g., near a pulmonary vein ostium) may serve as a backstop to advancement of a distal portion of the catheter (210). The distal portion of the catheter (210) may include a set of electrodes (212) (e.g., structurally and/or functionally similar to the electrode(s) (112)) configured to contact an inner radial surface of a lumen (e.g., pulmonary vein ostium). For example, the electrodes (212) may include an approximately circular arrangement of electrodes configured to contact a pulmonary vein ostium. As shown in FIG. 2, one or more electrodes (212) may include a series of metallic bands or rings disposed along a catheter shaft and be electrically connected together. For example, the ablation device (200) may comprise a single electrode having a plurality of bands, one or more electrodes each having its own band, and combinations thereof. In some embodiments, the electrodes (212) may be shaped to conform to the shape of the distal portion (222) of the guidewire (220). The catheter shaft may include flexible portions between the electrodes to enhance flexibility. In other embodiments, one or more electrodes (212) may include a helical winding to enhance flexibility.

Each of the electrodes of the ablation devices discussed herein is connected to an insulated electrical lead (not shown) leading to a handle (not shown) coupled to a proximal portion of the catheter. The insulation on each of the electrical leads can sustain an electrical potential difference of at least 700V across its thickness without dielectric breakdown. In other embodiments, the insulation on each of the electrical leads may sustain an electrical potential difference of up to about 2000 V across its thickness without dielectric breakdown, including all values and sub-ranges in between. This allows the electrodes to effectively deliver electrical energy and to ablate tissue through irreversible electroporation. The electrodes may, for example, receive pulse waveforms generated by a signal generator (122) as discussed above with respect to FIG. 1. In other embodiments, a guidewire (220) may be separate from the ablation device (200) (e.g., the ablation device (200) includes the catheter (210) but not the guidewire (220). For example, a guidewire (220) may be advanced by itself into an endocardial space, and thereafter the catheter (210) may be advanced into the endocardial space over the guidewire (220).

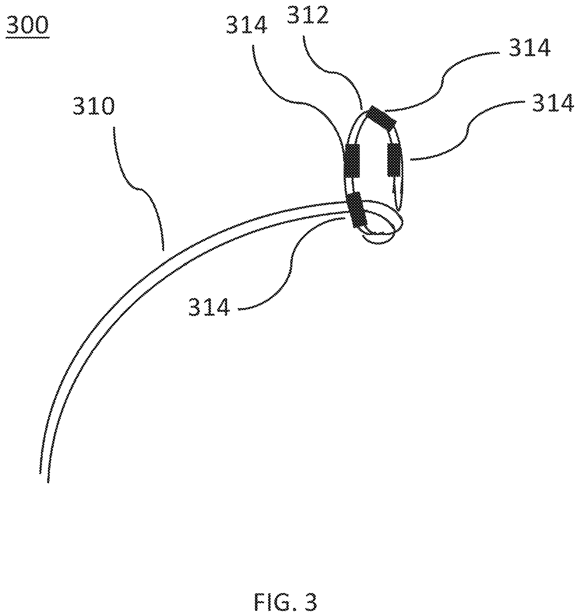

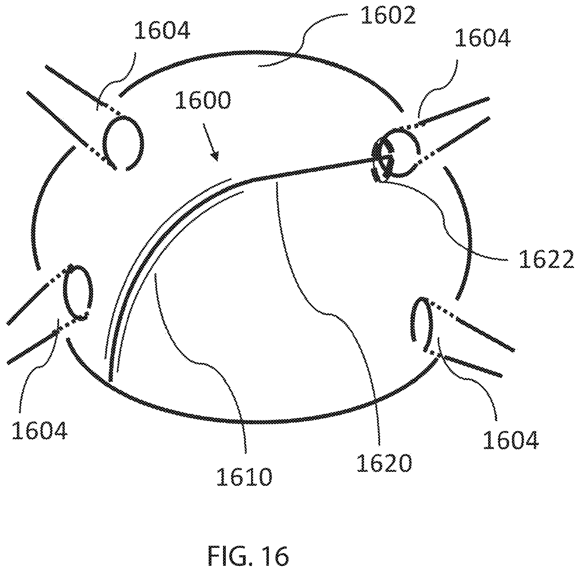

FIG. 3 is a perspective view of another ablation device (300) (e.g., structurally and/or functionally similar to the ablation device (110)) including a catheter (310) having a set of electrodes (314) provided along a distal portion (312) of the catheter (310). The distal portion (312) of the catheter (310) may be nonlinear and form an approximately circle shape. A set of electrodes (314) may be disposed along a nonlinear distal portion (312) of the catheter (310) may form a generally circular arrangement of electrodes (314). During use, the electrodes (314) may be disposed at a pulmonary vein ostium in order to deliver a pulse waveform to ablate tissue, as described in more detail with respect to FIG. 16. The shaped distal portion (312) of the catheter (310) may be angled relative to the other portions of the catheter (310). For example, the distal portion (312) of the catheter (310) may be generally perpendicular to an adjacent portion of the catheter (310). In some embodiments, a handle (not shown) may be coupled to a proximal portion of the catheter (310) and may include a bending mechanism (e.g., one or more pull wires (not shown)) configured to modify the shape of the distal portion (312) of the catheter (310). For example, operation of a pull wire of the handle may increase or decrease a circumference of the circular shape of the distal portion (312) of the catheter (310). The diameter of the distal portion (312) of the catheter (310) may be modified to allow the electrodes (314) to be disposed near and/or in contact with a pulmonary vein ostium (e.g., in contact with an inner radial surface of the pulmonary vein). The electrodes (314) may include a series of metallic bands or rings and be independently addressable.

In some embodiments, the pulse waveform may be applied between the electrodes (314) configured in anode and cathode sets. For example, adjacent or approximately diametrically opposed electrode pairs may be activated together as an anode-cathode set. It should be appreciated that any of the pulse waveforms disclosed herein may be progressively or sequentially applied over a sequence of anode-cathode electrodes.

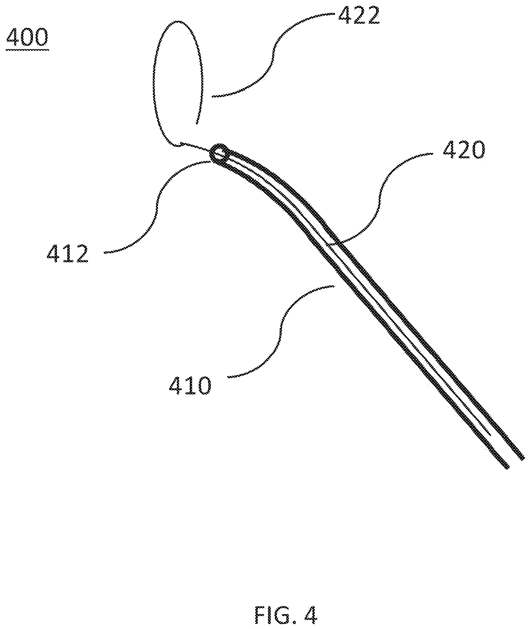

FIG. 4 is a perspective view of yet another ablation device (400) (e.g., structurally and/or functionally similar to the ablation device (110)) including a catheter (410) and a guidewire (420) having a shaped, nonlinear distal portion (422). The guidewire (420) may be slidable within a lumen of the catheter (410). The guidewire (420) may be advanced through the lumen of the catheter (410) and a distal portion (422) of the guidewire (420) may be approximately circular shaped. The shape and/or diameter of the distal portion (422) of the guidewire (420) may be modified using a bending mechanism as described above with respect to FIG. 3. The catheter (410) may be flexible so as to be deflectable. In some embodiments, the catheter (410) and/or guidewire (420) may be configured to be resilient such that they conform to a lumen in which they are disposed and assume a secondary shape when advanced out of the lumen. By modifying a size of the guidewire (420) and manipulating the deflection of the catheter (410), the distal portion (422) of the guidewire (420) may be positioned at a target tissue site, such as, a pulmonary vein ostium. A distal end (412) of the catheter (410) may be sealed off except where the guidewire (420) extends from such that the catheter (410) may electrically insulate the portion of the guidewire (420) within the lumen of the catheter (410). For example, in some embodiments, the distal end (412) of the catheter (410) may include a seal having an opening that permits passage of the guidewire (420) upon application of force to form a compression hold (that may be fluid-tight) between the seal and the guidewire (420).

In some embodiments, the exposed distal portion (422) of the guidewire (420) may be coupled to an electrode and configured to receive a pulse waveform from a signal generator and deliver the pulse waveform to tissue during use. For example, a proximal end of the guidewire (420) may be coupled to a suitable lead and connected to the signal generator (122) of FIG. 1. The distal portion (422) of the guidewire (420) may be sized such that it may be positioned at a pulmonary vein ostium. For example, a diameter of the shaped distal portion (422) of the guidewire (420) may be about the same as a diameter of a pulmonary vein ostium. The shaped distal portion (422) of the guidewire (420) may be angled relative to the other portions of the guidewire (420) and catheter (410).

The guidewire (420) may include stainless steel, nitinol, platinum, or other suitable, biocompatible materials. In some embodiments, the distal portion (422) of the guidewire (420) may include a platinum coil physically and electrically attached to the guidewire (420). The platinum coil may be an electrode configured for delivery of a voltage pulse waveform. Platinum is radiopaque and its use may increase flexibility to aid advancement and positioning of the ablation device (400) within an endocardial space.

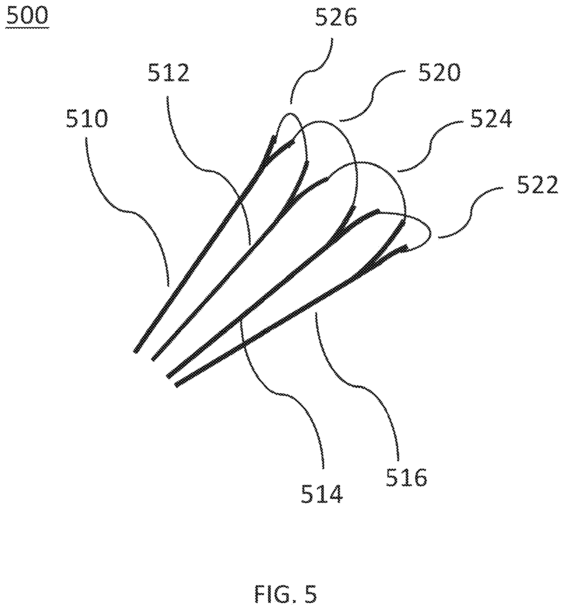

FIG. 5 is a detailed perspective view of a flower-shaped distal portion of an ablation device (500) (e.g., structurally and/or functionally similar to the ablation device (110)) including a set of electrodes (520, 522, 524, 526) each extending from a pair of insulated lead segments (510, 512, 514, 516). Each pair of adjacent insulated lead segments coupled to an uninsulated electrode (e.g., lead segments (510, 512) and electrode (526)) form a loop (FIG. 5 illustrates a set of four loops). The set of loops at the distal portion of the ablation device (500) may be configured for delivering a pulse waveform to tissue. The ablation device (500) may include a set of insulated lead segments (510, 5 12, 514, 516) that branch out at a distal end of the device (500) to connect to respective exposed electrodes (520, 522, 524, 526), as shown in FIG. 5. The electrodes (520, 522, 524, 526) may include an exposed portion of an electrical conductor. In some embodiments, one or more of the electrodes (520, 522, 524, 526) may include a platinum coil. The one or more segments (510, 512, 514, 516) may be coupled to a bending mechanism (e.g., strut, pull wire, etc.) controlled from a handle (not shown) to control a size and/or shape of the distal portion of the device (500).

The electrodes (520, 522, 524, 526) may be flexible and form a compact first configuration for advancement into an endocardial space, such as adjacent to a pulmonary vein ostium. Once disposed at a desired location, the electrodes (520, 522, 524, 526) may be transformed to an expanded second configuration when advanced out of a lumen, such as a sheath, to form a flower-shaped distal portion, as shown in FIG. 5. In other embodiments, the insulated lead segments (510, 512, 514, 516) and electrodes (520, 522, 524, 526) may be biased to expand outward (e.g., spring open) into the second configuration when advanced out of a lumen (e.g., sheath) carrying the device (500). The electrodes (520, 522, 524, 526) may be independently addressable and each have an insulated electrical lead configured to sustain a voltage potential of at least about 700 V without dielectric breakdown of its corresponding insulation. In other embodiments, the insulation on each of the electrical leads may sustain an electrical potential difference of between about 200 V to about 2000 V across its thickness without dielectric breakdown.

In some embodiments, the ablation device (5000 may be configured for delivering the pulse waveform to tissue during use via the set of electrodes (520, 522, 524, 526). In some embodiments, the pulse waveform may be applied between the electrodes (520, 522, 524, 526) configured in anode and cathode sets. For example, approximately diametrically opposite electrode pairs (e.g., electrodes (520, 524) and (522, 526)) may be activated together as an anode-cathode pair. In other embodiments, adjacent electrodes may be configured as an anode-cathode pair. As an example, a first electrode (520) of the set of electrodes may be configured as an anode and a second electrode (522) may be configured as a cathode.

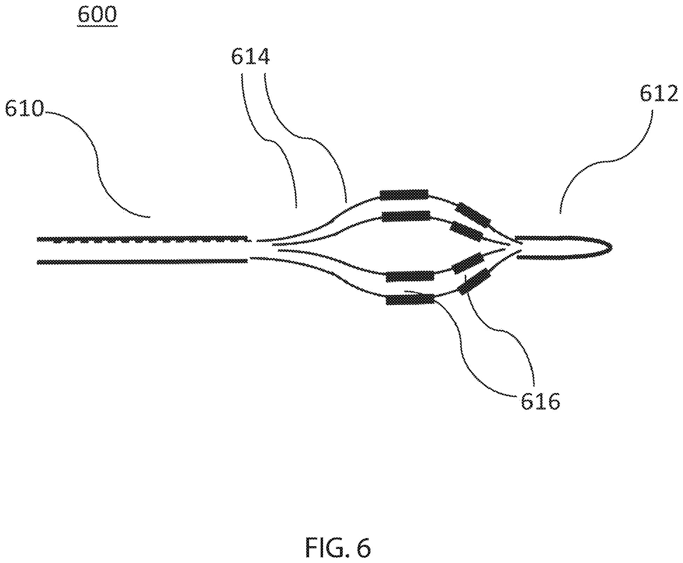

FIGS. 6-9E illustrate additional embodiments of an ablation device (e.g., structurally and/or functionally similar to the ablation device (110)) that may be configured to deliver voltage pulse waveforms using a set of electrodes to ablate tissue and electrically isolate a pulmonary vein. In some of these embodiments, the ablation device may be transformed from a first configuration to a second configuration such that the electrodes of the ablation device expand outward to contact a lumen of tissue (e.g., pulmonary vein ostium).

FIG. 6 is a side view of an ablation device (600) including a catheter shaft (610) at a proximal end of the device (600), a distal cap (612) of the device (600), and a set of spines (614) coupled thereto. The distal cap (612) may include an atraumatic shape to reduce trauma to tissue. A proximal end of the set of spines (614) may be coupled to a distal end of the catheter shaft (610), and a distal end of the set of spines (614) may be tethered to the distal cap (612) of the device (600). The ablation device (600) may be configured for delivering a pulse waveform to tissue during use via one or more spines of the set of spines (614).

Each spine (614) of the ablation device (600) may include one or more independently addressable electrodes (616) formed on a surface of the spine (614). Each electrode (616) may include an insulated electrical lead configured to sustain a voltage potential of at least about 700 V without dielectric breakdown of its corresponding insulation. In other embodiments, the insulation on each of the electrical leads may sustain an electrical potential difference of between about 200V to about 2000 V across its thickness without dielectric breakdown. Each spine (614) may include the insulated electrical leads of each electrode (616) formed in a body of the spine (614) (e.g., within a lumen of the spine (614)). FIG. 6 illustrates a set of spines (614) where each spine (614) includes a pair of electrodes (616) having about the same size, shape, and spacing as the electrodes (616) of an adjacent spine (614). In other embodiments, the size, shape, and spacing of the electrodes (616) may differ.

For each of the ablation devices described herein, and the ablation devices described in FIGS. 6-9 in particular, each spine of the set of spines may include a flexible curvature. The minimum radius of curvature of a spine can be in the range of about 1 cm or larger. For example, the set of spines may form a delivery assembly at a distal portion of the ablation device and be configured to transform between a first configuration where the set of spines bow radially outward from a longitudinal axis of the ablation device, and a second configuration where the set of spines are arranged generally parallel to the longitudinal axis of the ablation device. In this manner, the spines may more easily conform to the geometry of an endocardial space. In general, the "basket" of spines can have an asymmetric shape along the shaft length, so that one end (say the distal end) of the basket is more bulbous than the other end (say the proximal end) of the basket. The delivery assembly may be disposed in the first configuration in contact with the pulmonary vein ostium and transformed to the second configuration prior to delivering a pulse waveform. In some of these embodiments, a handle may be coupled to the set of spines and the handle configured for affecting transformation of the set of spines between the first configuration and the second configuration. In some embodiments, the electrical leads of at least two electrodes of the set of electrodes may be electrically coupled at or near a proximal portion of the ablation device, such as, for example, within the handle.

In one embodiment, each of the electrodes (616) on a spine (614) may be configured as an anode while each of the electrodes (616) on an adjacent spine (614) may be configured as a cathode. In another embodiment, the electrodes (616) on one spine may alternate between an anode and cathode with the electrodes of an adjacent spine having a reverse configuration (e.g., cathode and anode). The ablation device (600) may include any number of spines, for example, 3, 4, 5, 6, 7, 8, 9, 10, 12, 14, 16, 18, 20, or more spines, including all values and sub-ranges in between. In some embodiments, the ablation device (600) may include 3 to 20 spines. For example, the ablation device (600) may include 6 to 12 spines.

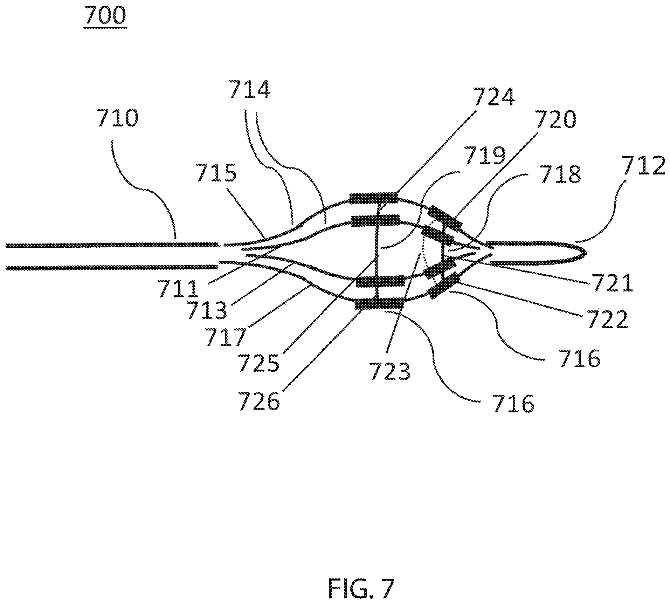

FIG. 7 is a side view of another ablation device (700) including a catheter shaft (710) at a proximal end of the device (700), a distal cap (712) of the device (700), and a set of spines (714) coupled thereto. The distal cap (712) may include an atraumatic shape. A proximal end of the set of spines (714) may be coupled to a distal end of the catheter shaft (710), and a distal end of the set of spines (714) may be tethered to the distal cap (712) of the device (700). Each spine (714) of the ablation device (700) may include one or more independently addressable electrodes (716) formed on a surface of the spine (714). Each electrode (716) may include an insulated electrical lead configured to sustain a voltage potential of at least about 700 V without dielectric breakdown of its corresponding insulation. In other embodiments, the insulation on each of the electrical leads may sustain an electrical potential difference of between about 200V to about 1500 V across its thickness without dielectric breakdown. Each spine (714) may include the insulated electrical leads of each electrode (716) formed in a body of the spine (714) (e.g., within a lumen of the spine (714)). A set of spine wires (718, 719) may be electrically conductive and electrically couple adjacent electrodes (716) disposed on different spines (714) such as electrodes (716) between a pair of spines (718, 719) of the set of spines. For example, the spine wires (718, 719) may extend in a transverse direction relative to a longitudinal axis of the ablation device (700).

FIG. 7 illustrates a set of spines (714) where each spine (714) includes a pair of electrodes (716) having about the same size, shape, and spacing as the electrodes (716) of an adjacent spine (714). In other embodiments, the size, shape, and spacing of the electrodes (716) may differ. For example, the electrodes (716) electrically coupled to a first spine wire (718) may differ in size and/or shape from electrodes (716) electrically coupled to a second spine wire (719).

In some embodiments, the first spine wire (718) may include a first set of spine wires (720, 721, 722, 723), where each spine wire of the set of spine wires (720, 721, 722, 723) may couple electrodes (716) between a different pair of spines of the set of spines (714). In some of these embodiments, the set of spine wires (720, 721, 722, 723) may form a continuous loop between the electrodes (716) coupled thereto. Likewise, the second spine wire (719) may include a second set of spine wires (724, 725, 726), where each spine wire of the set of spine wires (724, 725, 726) may couple electrodes (716) across the set of spines (714). The second set of spine wires (724, 725, 726) may couple different electrodes (716) across the set of spines (714) than the first set of spine wires (720, 721, 722, 723). In some of these embodiments, the first set of spine wires (720, 721, 722, 723) may form a first continuous loop between the electrodes (716) coupled thereto and the second set of spine wires (724, 725, 726) may form a second continuous loop between the electrodes (716) coupled thereto. The first continuous loop may be electrically isolated from the second continuous loop. In some of these embodiments, the electrodes (716) coupled to the first continuous loop may be configured as anodes and the electrodes (716) coupled to the second continuous loop may be configured as cathodes. A pulse waveform may be delivered to the electrodes (716) of the first and second continuous loop. In some embodiments, the spine wires such as 721, 722, 723 etc. can be replaced by similar electrical connections in the proximal part of the device (for example, in the device handle). For example, the electrodes 716 can all be electrically wired together in the handle of the device.

In another embodiment, the first spine wire (721) of the set of spine wires (720, 721, 722, 723) may couple electrodes (716) between a first spine (711) and a second spine (713) of the set of spines (714), and a second spine wire (720) of the set of spine wires (720, 721, 722, 723) may couple electrodes (716) between the first spine (711) and a third spine (715) of the set of spines (714). The electrodes (716) coupled by the first spine wire (721) and the second spine wire (720) may be configured as an anode and cathode (or vice-versa). In yet another embodiment, the first spine wire (721) of the set of spine wires (720, 721, 722, 723) may couple the electrodes (716) between a first spine (711) and a second spine (713) of the set of spines (714), and a second spine wire (723) of the set of spine wires (720, 721, 722, 723) may couple the electrodes (716) between a third spine (715) and a fourth spine (717) of the set of spines (714). A pulse waveform may be delivered to the electrodes (716) coupled by the first spine wire (721) and the second spine wire (723). In some embodiments, instead of spine wires the electrical leads of at least two electrodes of the set of electrodes are electrically coupled at or near a proximal portion of the ablation device, such as, for example, within a handle.

In other embodiments, one or more of the spine wires (718, 719) may form a continuous loop between the electrically coupled electrodes (716). For example, a first set of spine wires (718) may form a first continuous loop between the electrodes (716) coupled thereto and a second set of spine wires (719) may form a second continuous loop between the electrodes (716) coupled thereto. In this case, the first continuous loop may be electrically isolated from the second continuous loop. In one embodiment, each of the electrodes (716) coupled to a first set of spine wires (718) may be configured as an anode while each of the electrodes (716) coupled to a second set of spine wires (719) may be configured as a cathode. Each group of electrically coupled electrodes (716) may be independently addressable. In some embodiments, instead of spine wires the electrical leads of at least two electrodes of the set of electrodes are electrically coupled at or near a proximal portion of the ablation device, such as, for example, within a handle.

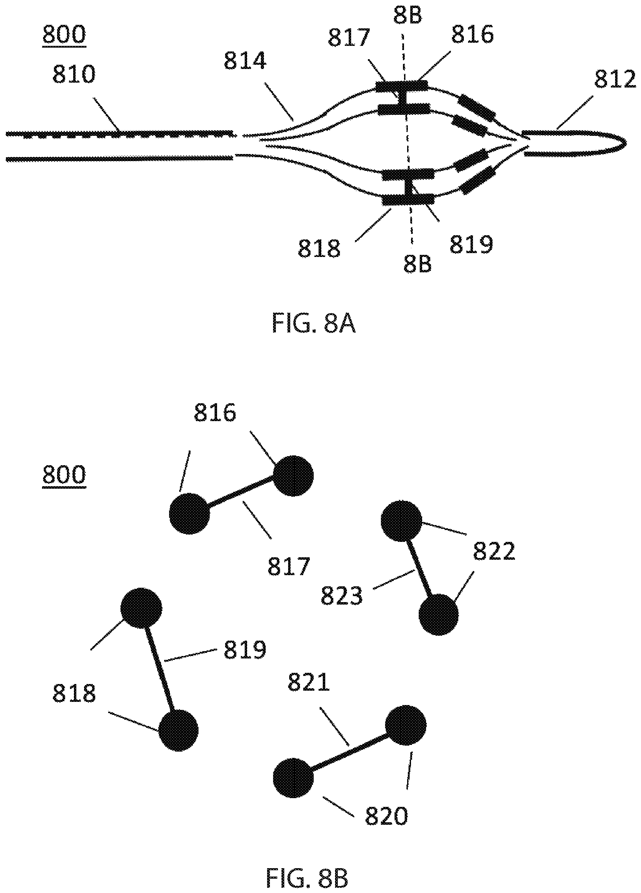

As discussed in further detail below with respect to FIGS. 8A-8B, a spine wire may electrically couple to a set of electrodes (e.g., 2, 3, 4, 5, etc.) without forming a continuous loop. For example, a discontinuous loop may be formed using two spine wires. In other embodiments, the size, shape, and spacing of the electrodes (716) may differ. The ablation device (700) may include any number of spines, for example, 3, 4, 5, 6, 7, 8, 9, 10, 12, 14, 16, 18, 20, or more spines. In some embodiments, the ablation device (700) may include 3 to 20 spines. For example, in one embodiment, the ablation device (700) may include 6 to 9 spines.

FIGS. 8A-8B are side and front cross-sectional views, respectively, of an ablation catheter (800). FIG. 8A is a side view of an ablation device (800) including a catheter shaft (810) at a proximal end of the device (800), a distal cap (812) of the device (800), and a set of spines (814) coupled thereto. The distal cap (812) may include an atraumatic shape. A proximal end of the set of spines (814) may be coupled to a distal end of the catheter shaft (810), and a distal end of the set of spines (14) may be tethered to the distal cap (812) of the device (800). Each spine (814) of the ablation device (800) may include one or more independently addressable electrodes (816, 818) formed on a surface of the spine (814). Each electrode (816, 818) may include an insulated electrical lead configured to sustain a voltage potential of at least about 700 V without dielectric breakdown of its corresponding insulation. In other embodiments, the insulation on each of the electrical leads may sustain an electrical potential difference of between about 200V to about 2000 V across its thickness without dielectric breakdown, including all values and sub-ranges in between. Each spine (814) may include the insulated electrical leads of each electrode (816, 818) formed in a body of the spine (814) (e.g., within a lumen of the spine (814)). One or more spine wires (817, 819) may be electrically conductive and electrically couple adjacent electrodes (816, 818) disposed on different spines (814). For example, the spine wires (817, 819) may extend in a transverse direction relative to a longitudinal axis of the ablation device (800).

FIG. 8B is a front cross-sectional view of FIG. 8A taken along the 8B-8B line. Each spine wire (817, 819, 821, 823) electrically couples a pair of adjacent electrodes (816, 818, 820, 822) on different spines. In some embodiments, each coupled electrode pair may be electrically isolated from each other. In some embodiments, the coupled electrode pair may be configured with a common polarity. Adjacent pairs of electrodes may be configured with opposite polarities (e.g., a first electrode pair configured as an anode and an adjacent second electrode pair configured as a cathode). For example, the electrodes (816) coupled to a first set of spine wires (817) may be configured as an anode while each of the electrodes (818) coupled to a second set of spine wires (819) may be configured as a cathode. In some embodiments, each electrode formed on a spine (814) may share a common polarity (e.g., configured as an anode or cathode). Each coupled electrode pair may be independently addressable. In some embodiments, the ablation device (800) may include an even number of spines. The ablation device (800) may include any number of spines, for example, 4, 6, 8, 10, or more spines. In some embodiments, the ablation device may include 4 to 10 spines. For example, in one embodiment, the ablation device may include 6 to 8 spines. As indicated in the foregoing, in some embodiments, the spine wires such as 817, 819, etc. can be replaced by similar electrical connections in the proximal part of the device (for example, in the device handle). For example, the electrodes 816 can be electrically wired together in the handle of the device, so that these electrodes are at the same electric potential during ablation.

FIG. 9A is a side view of yet another embodiment of an ablation device (900) including a catheter shaft (910) at a proximal end of the device (900), a distal cap (912) of the device (900), and a set of spines (914) coupled thereto. The distal cap (912) may include an atraumatic shape. A proximal end of the set of spines (914) may be coupled to a distal end of the catheter shaft (910), and a distal end of the set of spines (914) may be tethered to the distal cap (912) of the device (900). Each spine (914) of the ablation device (900) may include one or more independently addressable electrodes (916, 918) formed on a surface of the spine (914). Each electrode (916, 918) may include an insulated electrical lead configured to sustain a voltage potential of at least about 700 V without dielectric breakdown of its corresponding insulation. In other embodiments, the insulation on each of the electrical leads may sustain an electrical potential difference of up to about 2000 V across its thickness without dielectric breakdown. Each spine (914) may include the insulated electrical leads of each electrode (916, 918) formed in a body of the spine (914) (e.g., within a lumen of the spine (914)). FIG. 9A illustrates a set of spines (914) where each spine (914) includes an electrode (916, 918) spaced apart from an electrode (916, 918) of an adjacent spine (914). For example, the set of spines (914) including a first spine (920) and a second spine (922) adjacent to the first spine (920), wherein an electrode (916) of the first spine (920) is disposed closer to a distal end (912) of the ablation device (900) relative to an electrode (918) of the second spine (922). In other embodiments, the size and shape of the electrodes (916, 918) may differ as well.

In some embodiments, adjacent distal electrodes (916) and proximal electrodes (918) may form an anode-cathode pair. For example, the distal electrodes (916) may be configured as an anode and the proximal electrodes (918) may be configured as a cathode. In some embodiments, the ablation device (900) may include 6 to 12 spines. In FIG. 9A, one electrode (916, 918) is formed on a surface of each spine (914) such that each spine (914) includes one insulated electrical lead. A lumen of the spine (914) may therefore be reduced in diameter and allow the spine (914) to be thicker and more mechanically robust. Thus, dielectric breakdown of the insulation may be further reduced, thereby improving reliability and longevity of each spine (914) and the ablation device (900). The ablation device (900) may include any number of spines, for example, 3, 4, 5, 6, 7, 8, 9, 10, 12, 14, 16, 18, 20, or more spines. In some embodiments, the ablation device (900) may include 3 to 20 spines. For example, in one embodiment, the ablation device (900) may include 6 to 10 spines. Furthermore, in some embodiments, the shape of a bulb-like expanded structure (930) of the expanded set of spines (914) may be asymmetric, for example with its distal portion being more bulbous or rounded than its proximal portion (e.g., see FIGS. 9B-9E). Such a bulbous distal portion can aid in positioning the device at the ostium of a pulmonary vein.

Referring to FIGS. 9B-9E, it is understood that unless indicated otherwise, components with similar references numbers to those in FIG. 9A (e.g., the electrode (916) in FIG. 9A and the electrode (916') in FIG. 9B) may be structurally and/or functionally similar. FIG. 9B illustrates the spine wires (914', 920', 922') forming an expanded structure (930') during use such as when deployed. A first plane (924A'), also sometimes referred to as a proximal plane, of the expanded structure (930') has a cross-sectional area that is different than a cross-sectional area at a second plane (924B') of the expanded structure (930'). As illustrated in FIG. 9B, in some embodiments, the cross-sectional area of the expanded structure (930') at the second plane (924B') is greater than that at the first plane (924A'). The terms "first plane" and "second plane" as used with respect to FIG. 9B may refer to planes orthogonal to the longitudinal axis of the catheter shaft (910') that are each formed up to about 1 cm, about 2 cm, and about 3 cm or more (including all values and sub-ranges in between) from the distal end of the catheter shaft (910') and the proximal end of the distal cap (912'), respectively. Similar to FIG. 9A, the electrode (916') of the first spine (920') is disposed closer to the distal cap (912') of the ablation device (900') relative to an electrode (918') of the second spine (922').

FIG. 9C illustrates the spine wires (914", 920", 922") forming an expanded structure (930") during use such as when deployed. A first plane (924A"), also sometimes referred to as a proximal plane, of the expanded structure (930") has a cross-sectional area that is different than a cross-sectional area at a second plane (924B") of the expanded structure (930"). As illustrated in FIG. 9C, in some embodiments, the cross-sectional area of the expanded structure (930") at the second plane (924B") is greater than that at the first plane (924A"). The terms "first plane" and "second plane" as used with respect to FIG. 9C may refer to planes orthogonal to the longitudinal axis of the catheter shaft (910") that are each formed up to about 1 cm, about 2 cm, and about 3 cm or more (including all values and sub-ranges in between) from the distal end of the catheter shaft (910") and the proximal end of the distal cap (912"), respectively. Unlike FIGS. 9A-9B, multiple electrodes may be present on each spine wire, and some electrodes may be equidistant from the distal cap (912"), In this manner, relatively distal electrodes such as 932" and 934" may be apposed at or proximal/antral to a pulmonary vein ostium during use for ablation delivery to generate an ostial circumferential lesion around a pulmonary vein.

FIG. 9D illustrates the spine wires (914"', 920"', 922'") forming an expanded structure (930''') during use such as when deployed. The spine wires (914'", 920'", 922'") converge at their distal ends to a point (928 '") that lies inside/within the expanded structure (930'"). As illustrated in FIG. 9D, in such a configuration, at least some electrodes (932'", 934'") on the spine wires (914'", 920'", 922'") lie in a distal end plane (926'") of the expanded structure (930"'). The term "distal end plane" as used with respect to FIG. 9D refers to a plane orthogonal to the longitudinal axis of the catheter shaft (910'") that passes through a distal boundary of the expanded structure (930'"). In this manner, the expanded structure (930'") may be pressed against, for example, the posterior wall of the left atrium in order to directly generate lesions thereupon by activation of appropriate electrodes in the distal end plane using any suitable combination of polarities. For example, the electrodes 932"' and 934'" may be configured with opposite polarities.

FIG. 9E illustrates the spine wires (944, 940, 942) forming an expanded structure (950) during use such as when deployed. The spine wires (944, 940, 942) converge at their distal ends at a proximal end of a distal cap (912"") inside/within the expanded structure (950). As illustrated in FIG. 9E, in such a configuration, at least some electrodes (952, 954) on the spine wires (944, 940) may lie in a distal end plane (946) of the expanded structure (950). The term "distal end plane" as used with respect to FIG. 9E may refer to a plane orthogonal to the longitudinal axis of the catheter shaft (910"") that passes through a distal boundary of the expanded structure (950). In this manner, the expanded structure (950) may be pressed against, for example, the posterior wall of the left atrium in order to directly generate lesions thereupon by activation of appropriate electrodes in the distal end plane (946) using any suitable combination of polarities. For example, the electrodes 952 and 954 may be configured with opposite polarities. Relative to the expanded structure (930"") in FIG. 9D, the expanded structure (950) in FIG. 9E has a more orthogonal (e.g., flattened) shape that may be pressed against, for example, the posterior wall of the left atrium for tissue ablation. In other words, the cross-sectional area of the expanded structure (930"") at the distal end plane (926"") is less than that the cross-sectional area of the expanded structure (950) at the distal end plane (946).

For each of the ablation devices described herein, each of the spines may include a polymer and define a lumen so as to form a hollow tube. The one or more electrodes of the ablation device described herein may include a diameter from about 0.2 mm to about 2.0 mm and a length from about 0.2 mm to about 5.0 mm. In some embodiments, the electrode may include a diameter of about 1 mm and a length of about 1 mm. As the electrodes are independently addressable, the electrodes may be energized in any sequence using any pulse waveform sufficient to ablate tissue by irreversible electroporation. For example, different sets of electrodes may deliver different sets of pulses (e.g., hierarchical pulse waveforms), as discussed in further detail below. It should be appreciated that the size, shape, and spacing of the electrodes on and between the spines may be configured to deliver contiguous/transmural energy to electrically isolate one or more pulmonary veins. In some embodiments, alternate electrodes (for example, all the distal electrodes) can be at the same electric potential, and likewise for all the other electrodes (for example, all the proximal electrodes). Thus ablation can be delivered rapidly with all electrodes activated at the same time. A variety of such electrode pairing options exist and may be implemented based on the convenience thereof. Balloon (not part of the invention)

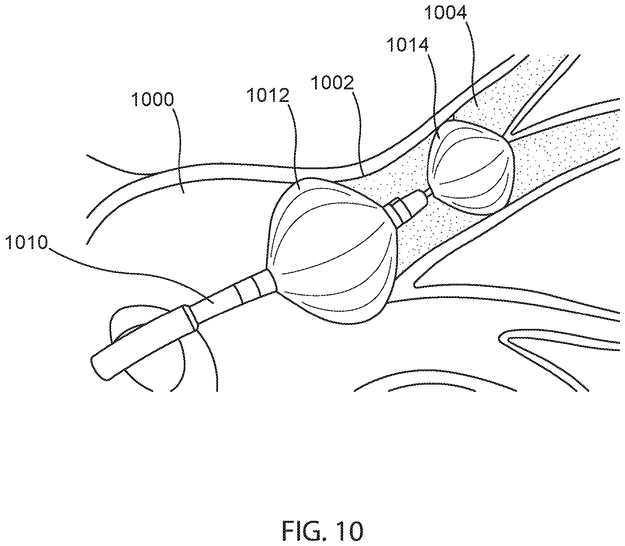

In some examples, not forming part of the invention, an ablation device may include one or more balloons for delivering energy to ablate tissue by irreversible electroporation. FIG. 10 depicts an embodiment of a balloon ablation device (1010) (e.g., structurally and/or functionally similar to the ablation device (110)) disposed in a left atrial chamber (1000) of a heart. The ablation device (1010) may include a first balloon (1012) and a second balloon (1014) which may be configured to be disposed in an ostium (1002) of a pulmonary vein (1004). The first balloon (1012) in an expanded (e.g., inflated) configuration may have a larger diameter than the second balloon (1014) in an expanded configuration. This allows the second balloon (1014) to be advanced and disposed further into the pulmonary vein (1014) while the first balloon (1012) may be disposed near and/or at an ostium (1002) of the pulmonary vein (1004). The inflated second balloon serves to stabilize the positioning of the first balloon at the ostium of the pulmonary vein. In some embodiments, the first balloon (1012) and the second balloon (1014) may be filled with any suitable conducting fluid such as, for example, saline. The first balloon (1012) and the second balloon (1014) may be electrically isolated from each other. For example, each balloon (1012, 1014) may include an insulated electrical lead associated therewith, with each lead having sufficient electrical insulation to sustain an electrical potential difference of at least 700V across its thickness without dielectric breakdown. In other embodiments, the insulation on each of the electrical leads may sustain an electrical potential difference of between about 200 V to about 2500 V across its thickness without dielectric breakdown, including all values and sub-ranges in between. For example, a lead of the second balloon (1014) may be insulated as it extends through the first balloon (1012).

The first and second balloons (1012, 1014) may form an anode-cathode pair. For example, the first balloon (1012) may be configured as a cathode and the second balloon (1014) may be configured as an anode, or vice versa, where electrical energy may be capacitively coupled across the balloon or saline-filled electrodes. The device (1010) may receive a pulse waveform to be delivered to tissue (1002). For example, one or more of a biphasic signal and monophasic signal may be applied such that tissue may be ablated between the first balloon (1012) and the second balloon (1014) at a desired location in the pulmonary vein (1004). The first and second balloons (1012, 1014) may confine the electric field substantially between the first and second balloons (1012, 1014) so as to reduce the electric field and damage to tissue away from the ostium (1002) of the pulmonary vein (1004). In some embodiments, one or more of the balloons (1012, 1014) may include a wire mesh.

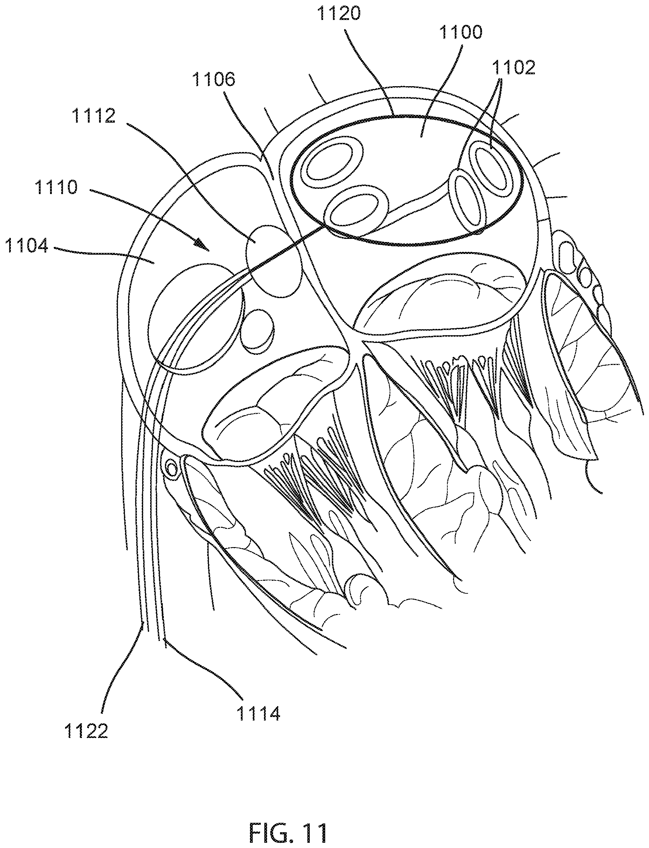

FIG. 11 is a cross-sectional view of another balloon ablation device (1110) (e.g., structurally and/or functionally similar to the ablation device (110)) disposed in a left atrial chamber (1100) and a right atrial chamber (1104) of a heart. The ablation device (1110) may include a balloon (1112) which may be configured to be advanced into and disposed in the right atrial chamber (1104). For example, the balloon (1112) may be disposed in contact with a septum (1106) of the heart. The balloon (1112) may be filled with saline. The device (1110) may further include an electrode (1120) that may be advanced from the right atrial chamber (1104) through the balloon (1112) and the septum (1106) and into the left atrial chamber (1100). For example, the electrode (1120) may extend from the balloon (1112) and puncture through the septum (1106) and be advanced into the left atrial chamber (1100). Once the electrode (1120) is advanced into the left atrial chamber (1100), a distal portion of the electrode (1120) may be modified to form a predetermined shape. For example, a distal portion of the electrode (1120) may include a nonlinear shape such as a circle, ellipsoid, or any other geometric shape. In FIG. 11, the distal portion of the electrode (1120) forms a loop that may surround a single ostium or two or more ostia of the pulmonary veins (1102) in the left atrial chamber (1100). The distal portion of the electrode (1120) may have about the same diameter as an ostium of the pulmonary vein (1102).

The balloon (1112) and the electrode (1120) may be electrically isolated from each other. For example, the balloon (1112) and the electrode (1120) may each include an insulated electrical lead (1114, 1122) respectively, with each lead (1114, 1122) having sufficient electrical insulation to sustain an electrical potential difference of at least 700V across its thickness without dielectric breakdown. The insulation on each of the electrical leads may sustain an electrical potential difference of between about 200 V to about 2,000 V across its thickness without dielectric breakdown, including all values and sub-ranges in between. The lead (1122) of the electrode (1120) may be insulated through the balloon (1112). The balloon (1112) and the electrode (1120) may form an anode-cathode pair. For example, the balloon (1112) may be configured as a cathode and the electrode (1120) may be configured as an anode. The device (1110) may receive a pulse waveform to be delivered to the ostium of the pulmonary veins (1102). For example, one or more of a biphasic signal and monophasic signal may be applied to ablate tissue. The pulse waveform may create an intense electric field around the electrode (1120) while the current applied is capacitively coupled to the balloon (1112) to complete the circuit. The electrode (1120) may include a fine gauge wire and the balloon (1112) may include a wire mesh.

The electrode (1120) may be advanced through the pulmonary veins (1102) and disposed in one or more of the pulmonary vein ostia without being advanced through the balloon (1112) and/or the septum (1106). The balloon (1112) and electrode (1120) may be configured as a cathode-anode pair and receive a pulse waveform in the same manner as discussed above.

### Return Electrode

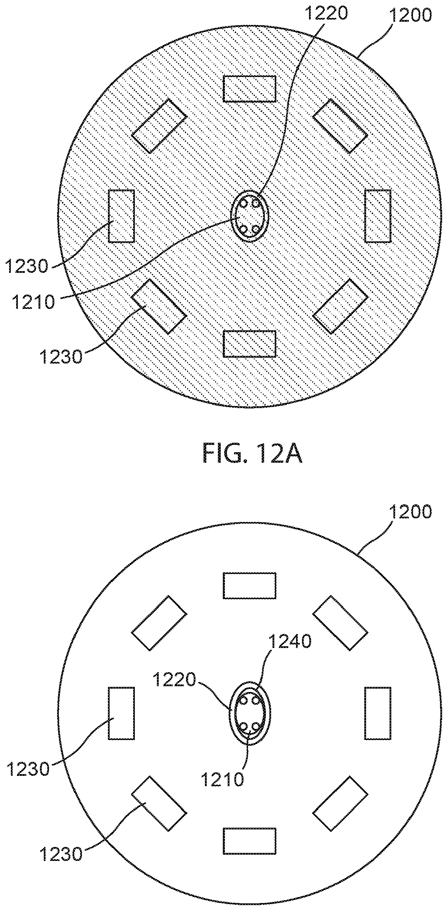

Some embodiments of an ablation system as described herein may further include a return electrode or a distributed set of return electrodes coupled to a patient to reduce the risk of unintended damage to healthy tissue. FIGS. 12A-12B are schematic views of a set of return electrodes (1230) (e.g., return pad) of an ablation system disposed on a patient (1200). A set of four ostia of the pulmonary veins (1210) of the left atrium are illustrated in FIGS. 12A-12B. An electrode (1220) of an ablation device may be positioned around one or more of the ostia of the pulmonary veins (1210). In some embodiments, a set of return electrodes (1230) may be disposed on a back of a patient (1200) to allow current to pass from the electrode (1220) through the patient (1200) and then to the return electrode (1230).

For example, one or more return electrodes may be disposed on a skin of a patient (1200). In one embodiment, eight return electrodes (1230) may be positioned on the back of the patient so as to surround the pulmonary vein ostia. (1210). A conductive gel may be applied between the return electrodes (1230) and the skin to improve contact. It should be appreciated that any of the ablation devices described herein may be used with the one or more return electrodes (1230). In FIGS. 12A-12B, the electrode (1220) is disposed around four ostia (1210).

FIG. 12B illustrates the energized electrode (1220) forming an electric field (1240) around the ostia (1210) of the pulmonary veins. The return electrode (1230) may in turn receive a pulsed monophasic and/or biphasic waveform delivered by the electrode (1220). In some embodiments, the number of return electrodes (1230) may be inversely proportional to the surface area of the return electrodes (1230).

For each of the ablation devices discussed herein, the electrodes (e.g., ablation electrode, return electrode) may include biocompatible metals such as titanium, palladium, silver, platinum or a platinum alloy. For example, the electrode may preferably include platinum or a platinum alloy. Each electrode includes an electrical lead having sufficient electrical insulation to sustain an electrical potential difference of at least 700V across its thickness without dielectric breakdown. In other embodiments, the insulation on each of the electrical leads may sustain an electrical potential difference of between about 200V to about 2500 V across its thickness without dielectric breakdown, including all values and sub-ranges in between. The insulated electrical leads may run to the proximal handle portion of the catheter from where they may be connected to a suitable electrical connector. The catheter shaft may be made of a flexible polymeric material such as Teflon, Nylon, Pebax, etc.

### II. Methods (not part of the invention)

Also described here are methods for ablating tissue in a heart chamber using the systems and devices described above. The heart chamber may be the left atrial chamber and include its associated pulmonary veins. Generally, the methods described here include introducing and disposing a device in contact with one or more pulmonary vein ostia. A pulse waveform may be delivered by one or more electrodes of the device to ablate tissue. In some embodiments, a cardiac pacing signal may synchronize the delivered pulse waveforms with the cardiac cycle. Additionally or alternatively, the pulse waveforms may include a plurality of levels of a hierarchy to reduce total energy delivery. The tissue ablation thus performed may be delivered in synchrony with paced heartbeats and with less energy delivery to reduce damage to healthy tissue. It should be appreciated that any of the ablation devices described herein may be used to ablate tissue using the methods discussed below as appropriate.

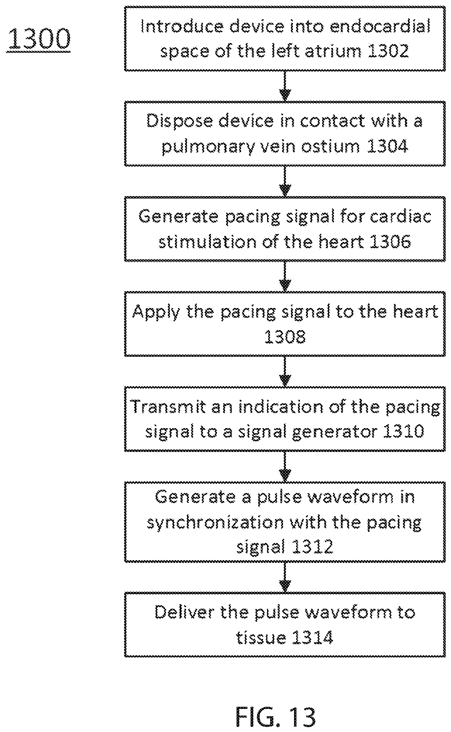

FIG. 13 is a method (1300) for one embodiment of a tissue ablation process. In some embodiments, the voltage pulse waveforms described herein may be applied during a refractory period of the cardiac cycle so as to avoid disruption of the sinus rhythm of the heart. The method (1300) includes introduction of a device (e.g., ablation device, such as the ablation device (110), and/or any of the ablation devices (200, 300, 400, 500, 600, 700, 800, 900, 1010, 1110) into an endocardial space of a left atrium at step (1302). The device may be advanced to be disposed in contact with a pulmonary vein ostium (1304). For example, electrodes of an ablation device may form an approximately circular arrangement of electrodes disposed in contact with an inner radial surface at a pulmonary vein ostium. In some embodiments, a pacing signal may be generated for cardiac stimulation of the heart (1306). The pacing signal may then be applied to the heart (1308). For example, the heart may be electrically paced with a cardiac stimulator to ensure pacing capture to establish periodicity and predictability of the cardiac cycle. One or more of atrial and ventricular pacing may be applied. An indication of the pacing signal may be transmitted to a signal generator (1310). A time window within the refractory period of the cardiac cycle may then be defined within which one or more voltage pulse waveforms may be delivered. In some embodiments, a refractory time window may follow a pacing signal. For example, a common refractory time window may lie between both atrial and ventricular refractory time windows.

A pulse waveform may be generated in synchronization with the pacing signal (1312). For example, a voltage pulse waveform may be applied in the common refractory time window. In some embodiments, the pulse waveform may be generated with a time offset with respect to the indication of the pacing signal. For example, the start of a refractory time window may be offset from the pacing signal by a time offset. The voltage pulse waveform(s) may be applied over a series of heartbeats over corresponding common refractory time windows. The generated pulse waveform may be delivered to tissue (1314). In some embodiments, the pulse waveform may be delivered to pulmonary vein ostium of a heart of a patient via one or more spines of a set of spines of an ablation device. In other embodiments, voltage pulse waveforms as described herein may be selectively delivered to electrode subsets such as anode-cathode subsets for ablation and isolation of the pulmonary vein. For example, a first electrode of a group of electrodes may be configured as an anode and a second electrode of the group of electrodes may be configured as a cathode. These steps may be repeated for a desired number of pulmonary vein ostium have been ablated (e.g., 1, 2, 3, or 4 ostia).

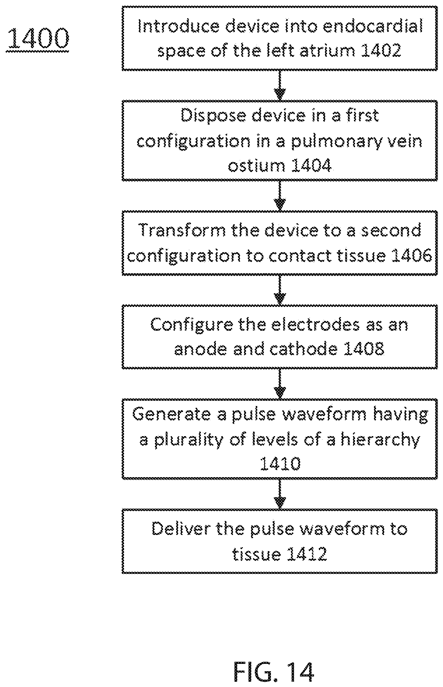

In some embodiments, hierarchical voltage pulse waveforms having a nested structure and a hierarchy of time intervals as described herein may be useful for irreversible electroporation, providing control and selectivity in different tissue types. FIG. 14 is a flowchart (1400) of another embodiment of a tissue ablation process. The method (1400) includes the introduction of a device (e.g., ablation device, such as the ablation device (110) and/or any of the ablation devices (200, 300, 400, 500, 600, 700, 800, 900, 1010, 1110) into an endocardial space of a left atrium (1402). The device may be advanced to be disposed in a pulmonary vein ostium (1404). In embodiments where the device may include a first and second configuration (e.g., compact and expanded), the device may be introduced in the first configuration and transformed to a second configuration to contact tissue at or near the pulmonary vein ostium (1406). The device may include electrodes and may be configured in anode-cathode subsets (1408) as discussed in detail above. For example, a subset of electrodes of the devices may be selected as anodes, while another subset of electrodes of the device may be selected as cathodes, with the voltage pulse waveform applied between the anodes and cathodes.

A pulse waveform may be generated by a signal generator (e.g., the signal generator 122) and may include a plurality of levels in a hierarchy (1410). A variety of hierarchical waveforms may be generated with a signal generator as disclosed herein. For example, the pulse waveform may include a first level of a hierarchy of the pulse waveform including a first set of pulses. Each pulse has a pulse time duration and a first time interval separating successive pulses. A second level of the hierarchy of the pulse waveform may include a plurality of first sets of pulses as a second set of pulses. A second time interval may separate successive first sets of pulses. The second time interval may be at least three times the duration of the first time interval. A third level of the hierarchy of the pulse waveform may include a plurality of second sets of pulses as a third set of pulses. A third time interval may separate successive second sets of pulses. The third time interval may be at least thirty times the duration of the second level time interval.

It is understood that while the examples herein identify separate monophasic and biphasic waveforms, it should be appreciated that combination waveforms, where some portions of the waveform hierarchy are monophasic while other portions are biphasic, may also be generated. A voltage pulse waveform having a hierarchical structure may be applied across different anode-cathode subsets (optionally with a time delay). As discussed above, one or more of the waveforms applied across the anode-cathode subsets may be applied during the refractory period of a cardiac cycle. The pulse waveform may be delivered to tissue (1412). It should be appreciated that the steps described in FIGS. 13 and 14 may be combined and modified as appropriate.

FIGS. 15-18 depict methods for ablating tissue in a left atrial chamber of the heart as described above using the ablation devices described herein (e.g., FIGS. 2-5). FIG. 15 is a cross-sectional view of a method to ablate tissue disposed in a left atrial chamber of a heart using an ablation device (1500) corresponding to the ablation device (210) depicted in FIG. 2. The left atrial chamber (1502) is depicted having four pulmonary veins (1504) and the ablation device (1500) may be used to ablate tissue sequentially to electrically isolate one or more of the pulmonary veins (1504). As shown in FIG. 15, the ablation device (1500) may be introduced into an endocardial space such as the left atrial chamber (1502) using a trans-septal approach (e.g., extending from a right atrial chamber through the septum and into the left atrial chamber (1502)). The ablation device (1500) may include a catheter (1510) and a guide wire (1520) slidable within a lumen of the catheter (1510). A distal portion of the catheter (1510) may include a set of electrodes (1512). A distal portion (1522) of the guidewire (1520) may be advanced into the left atrial chamber (1502) so as to be disposed near an ostium of a pulmonary vein (1504). The catheter (1510) may then be advanced over the guidewire (1520) to dispose the electrodes (1512) near the ostium of the pulmonary vein (1504). Once the electrodes (1512) are in contact with the ostium of the pulmonary vein (1504), the electrodes (1512) may be configured in anode-cathode subsets. A voltage pulse waveform generated by a signal generator (not shown) may be delivered to tissue using the electrodes (1512) in synchrony with paced heartbeats and/or include a waveform hierarchy. After completion of tissue ablation in one of the pulmonary veins (1504), the catheter (1510) and guidewire (1520) may be repositioned at another pulmonary vein (1504) to ablate tissue in one or more of the remaining pulmonary veins (1504).

FIG. 16 is a cross-sectional view of a method to ablate tissue disposed in a left atrial chamber of a heart using an ablation device (1600) corresponding to the ablation device (310) depicted in FIG. 3. The left atrial chamber (1602) is depicted having four pulmonary veins (1604) and the ablation device (1600) may be used to ablate tissue sequentially to electrically isolate one or more of the pulmonary veins (1604). As shown in FIG. 16, the ablation device (1600) may be introduced into an endocardial space such as the left atrial chamber (1602) using a trans-septal approach. The ablation device (1600) may include a sheath (1610) and a catheter (1620) slidable within a lumen of the sheath (1610). A distal portion (1622) of the catheter (1620) may include a set of electrodes. A distal portion (1622) of the catheter (1620) may be advanced into the left atrial chamber (1602) to dispose the electrodes near an ostium of a pulmonary vein (1604). Once the electrodes are in contact with the ostium of the pulmonary vein (1604), the electrodes may be configured in anode-cathode subsets. A voltage pulse waveform generated by a signal generator (not shown) may be delivered to tissue using the electrodes in synchrony with paced heartbeats and/or include a waveform hierarchy. After completion of tissue ablation in the pulmonary vein (1604), the catheter (1620) may be repositioned at another pulmonary vein (1604) to ablate tissue in one or more of the remaining pulmonary veins (1604).

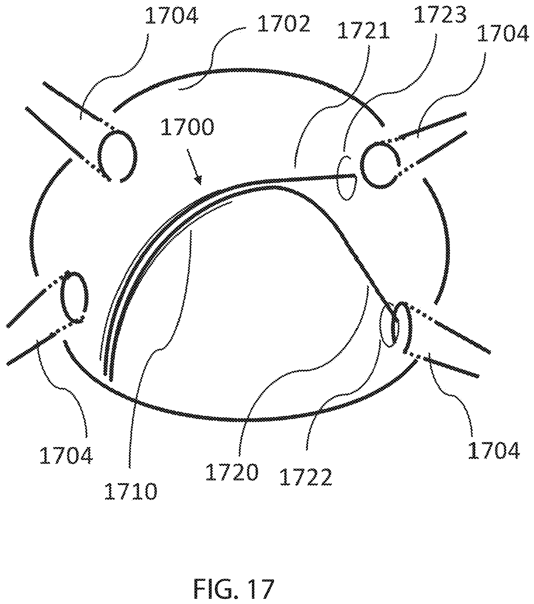

FIG. 17 is a cross-sectional view of a method to ablate tissue disposed in a left atrial chamber of a heart using an ablation device corresponding to the ablation device (410) depicted in FIG. 4. The left atrial chamber (1702) is depicted having four pulmonary veins (1704) and the ablation device (1700) may be used to ablate tissue to electrically isolate one or more of the pulmonary veins (1704). As shown in FIG. 17, the ablation device (1700) may be introduced into an endocardial space such as the left atrial chamber (1702) using a trans-septal approach. The ablation device (1700) may include a sheath (1710) and a plurality of catheters (1720, 1721) slidable within a lumen of the sheath (1710). Each of the catheters (1720, 1721) may include a respective guidewire (1722, 1723) slidable within the catheter (1720, 1721). A distal portion of the guidewire (1722, 1723) may include an electrode configured to deliver a voltage pulse waveform. Each of the catheters (1720, 1721) and corresponding guidewires (1722, 1723) may be advanced into the left atrial chamber (1702) so as to be disposed near respective ostia of the pulmonary veins (1704). Once the guidewire electrodes (1722, 1723) are in contact with the ostium of the pulmonary vein (1704), the electrodes may be configured in anode-cathode subsets. For example, a first guidewire (1722) may be configured as an anode while a second guidewire (1723) may be configured as a cathode. In this configuration, voltage pulse waveforms generated by a signal generator (not shown) may be delivered for ablation and simultaneous isolation of the pair of pulmonary veins (1704). Additionally or alternatively, a voltage pulse waveform may be delivered to tissue using the electrodes in synchrony with paced heartbeats and/or include a waveform hierarchy. After completion of tissue ablation in two of the pulmonary veins (1704), the catheters (1720, 1721) may be repositioned to ablate tissue at the two remaining pulmonary veins (1704). In some embodiments, the sheath (1710) may include three or four catheters to be disposed in the pulmonary veins (1704).

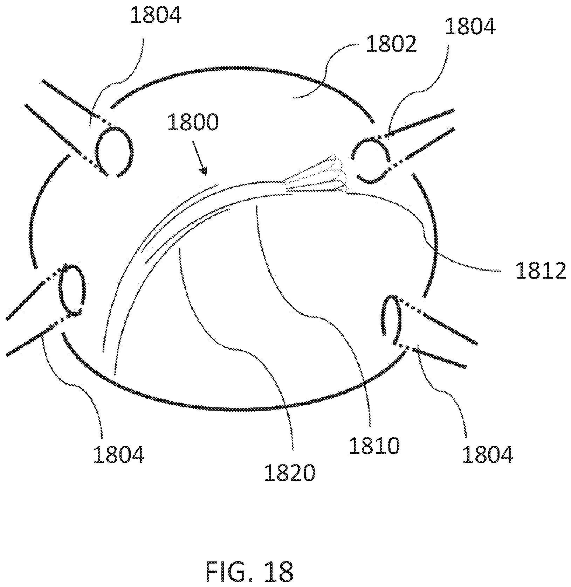

FIG. 18 is a cross-sectional view of a method to ablate tissue disposed in a left atrial chamber of a heart using an ablation device (1800) corresponding to the ablation device (500) depicted in FIG. 5. The left atrial chamber (1802) is depicted having four pulmonary veins (1804) and the ablation device (1800) may be used to ablate tissue sequentially to electrically isolate one or more of the pulmonary veins (1804). As shown in FIG. 18, the ablation device may be introduced into an endocardial space such as the left atrial chamber (1802) using a trans-septal approach. The ablation device may include a sheath (1820) and a catheter (1810) slidable within a lumen of the sheath (1820). A distal portion (1812) of the catheter (1810) may be flower-shaped as discussed in detail with respect to FIG. 5. A distal portion (1812) of the catheter (1810) may be advanced into the left atrial chamber (1802) in a compact first configuration and disposed near an ostium of a pulmonary vein (1804). The distal portion (1812) of the catheter (1810) may then be transformed to an expanded second configuration to form a flower-shaped distal portion, as shown in FIG. 18, such that the distal portion (1812) of the catheter (1810) is disposed near the ostium of the pulmonary vein (1804). Once the electrodes are in contact with the ostium of the pulmonary vein (1804), the electrodes may be configured in anode-cathode subsets. A voltage pulse waveform generated by a signal generator (not shown) may be delivered to tissue using the electrodes in synchrony with paced heartbeats and/or include a waveform hierarchy. After completion of tissue ablation in the pulmonary vein (1804), the catheter (1810) may be repositioned at another pulmonary vein (1804) to ablate tissue in one or more of the remaining pulmonary veins (1804).

It should be appreciated that any of the methods described herein (e.g., FIGS. 13-18) may further include coupling a return electrode (e.g., one or more return electrodes (1230) depicted in FIGS. 12A-12B) to a patient's back and configured to safely remove current from the patient during application of a voltage pulse waveform.

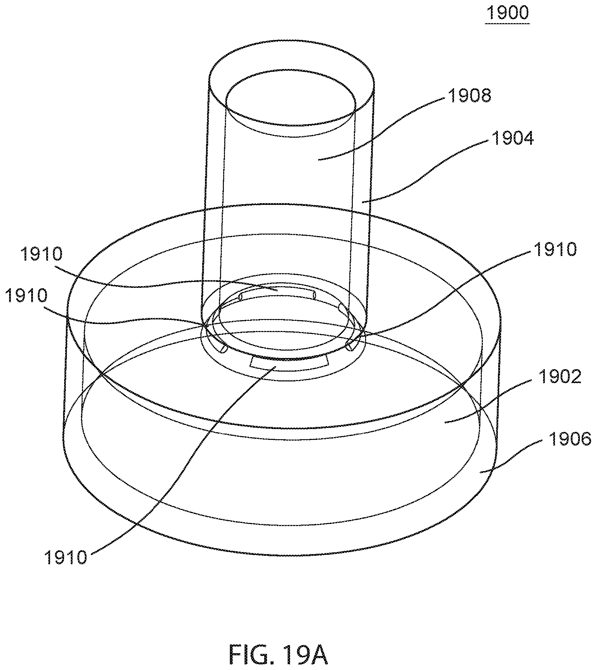

FIGS. 19A-20B depict electrodes disposed in contact around an ostium of a pulmonary vein and electric fields generated therefrom. FIG. 19A is a schematic representation (1900) of a set of electrodes (1910) disposed in an ostium of a pulmonary vein (1904). A left atrial chamber (1902) may include a blood pool (1906) and the pulmonary vein (1904) may include a blood pool (1908). The left atrial chamber (1902) and pulmonary vein (1904) may each have a wall thickness of up to about 4 mm.

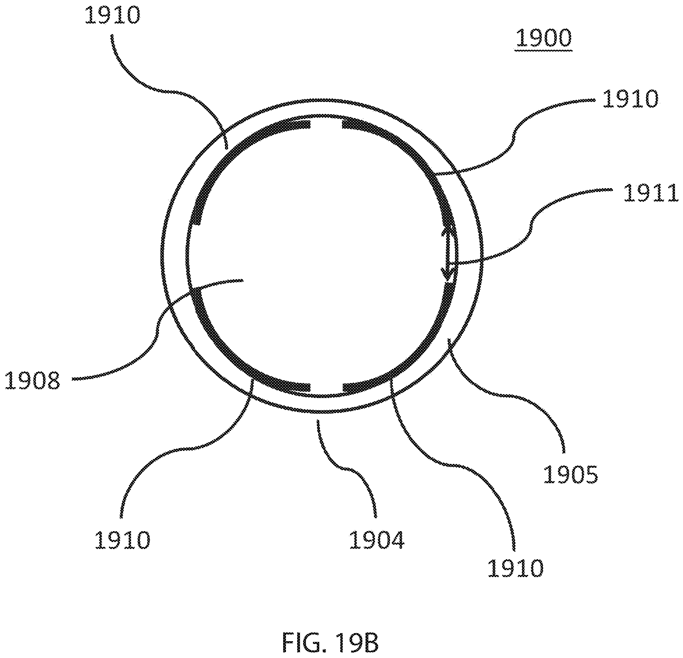

FIG. 19B is another schematic representation (1900) of the set of electrodes (1910) disposed radially along an interior surface of a pulmonary vein (1904). The pulmonary vein (1904) may include an arterial wall (1905) containing a blood pool (1908). Adjacent electrodes (1910) may be separated by a predetermined distance (1911). The pulmonary vein (1904) may have an inner diameter of about 16 mm. In FIGS. 19A-19B, the electrodes (1910) may have a length of about 10 mm and be spaced apart about 4 mm from each other. It should be appreciated that the electrodes (1910) may be any of the electrodes disclosed herein. For example, the electrodes (1910) may include the electrodes of the flower-shaped distal portion of FIG. 5 and/or the generally circular arrangement of electrodes depicted in FIG. 3.

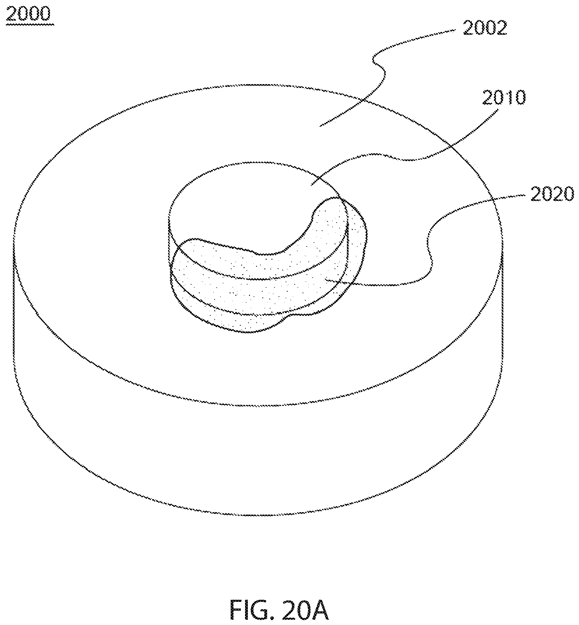

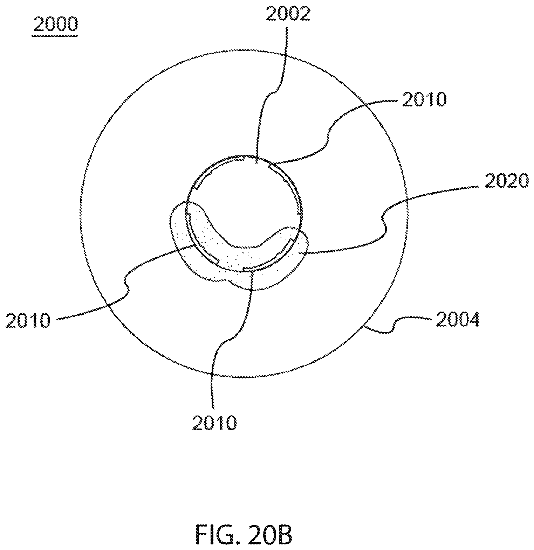

FIGS. 20A-20B are schematic representations (2000) of an electric field (2020) generated by a set of electrodes (2010) disposed in an ostium of a pulmonary vein (2002). FIG. 20A is a perspective view while FIG. 20B is a cross-sectional view of the pulmonary vein (2002) and outer wall of the left atrial chamber (2004). The shaded electric field (2020) illustrates where the electric field (2020) exceeds a threshold value when adjacent electrodes (2010) deliver energy (e.g., voltage pulse waveform) to ablate tissue. For example, the electric field (2020) represents a potential difference of 1500 V applied between adjacent electrodes (2010). Under this applied voltage, the electric field (2020) magnitude is at least above a threshold value of 500 V/cm within the shaded volumetric electric field (2020) and may be sufficient to generate irreversible ablation in cardiac tissue. By sequencing pulse waveforms over adjacent pairs of electrodes (2010) as described above in detail, a pulmonary vein (2002) ostium may be ablated to electrically isolate the pulmonary vein (2002) from the left atrial chamber (2004).

### Pulse Waveform

Disclosed herein are methods, systems and apparatuses for the selective and rapid application of pulsed electric fields/waveforms to effect tissue ablation with irreversible electroporation. The pulse waveform(s) as disclosed herein are usable with any of the systems (100), devices (e.g., 200, 300, 400, 500, 600, 700, 800, 900, 1010, 1110, 1230, 1500, 1600, 1700, 1800, 1910, 2010), and methods (e.g., 1300, 1400) described herein. Some embodiments are directed to pulsed high voltage waveforms together with a sequenced delivery scheme for delivering energy to tissue via sets of electrodes. In some embodiments, peak electric field values can be reduced and/or minimized while at the same time sufficiently large electric field magnitudes can be maintained in regions where tissue ablation is desired. This also reduces the likelihood of excessive tissue damage or the generation of electrical arcing, and locally high temperature increases. In some embodiments, a system useful for irreversible electroporation includes a signal generator and a processor capable of being configured to apply pulsed voltage waveforms to a selected plurality or a subset of electrodes of an ablation device. In some embodiments, the processor is configured to control inputs whereby selected pairs of anode-cathode subsets of electrodes can be sequentially triggered based on a pre-determined sequence, and in one embodiment the sequenced delivery can be triggered from a cardiac stimulator and/or pacing device. In some embodiments, the ablation pulse waveforms are applied in a refractory period of the cardiac cycle so as to avoid disruption of the sinus rhythm of the heart. One example method of enforcing this is to electrically pace the heart with a cardiac stimulator and ensure pacing capture to establish periodicity and predictability of the cardiac cycle, and then to define a time window well within the refractory period of this periodic cycle within which the ablation waveform is delivered.

In some embodiments, the pulsed voltage waveforms disclosed herein are hierarchical in organization and have a nested structure. In some embodiments, the pulsed waveform includes hierarchical groupings of pulses with a variety of associated timescales. Furthermore, the associated timescales and pulse widths, and the numbers of pulses and hierarchical groupings, can be selected so as to satisfy one or more of a set of Diophantine inequalities involving the frequency of cardiac pacing.

Pulsed waveforms for electroporation energy delivery as disclosed herein may enhance the safety, efficiency and effectiveness of the energy delivery by reducing the electric field threshold associated with irreversible electroporation, yielding more effective ablative lesions with reduced total energy delivered. This in turn can broaden the areas of clinical application of electroporation including therapeutic treatment of a variety of cardiac arrhythmias.

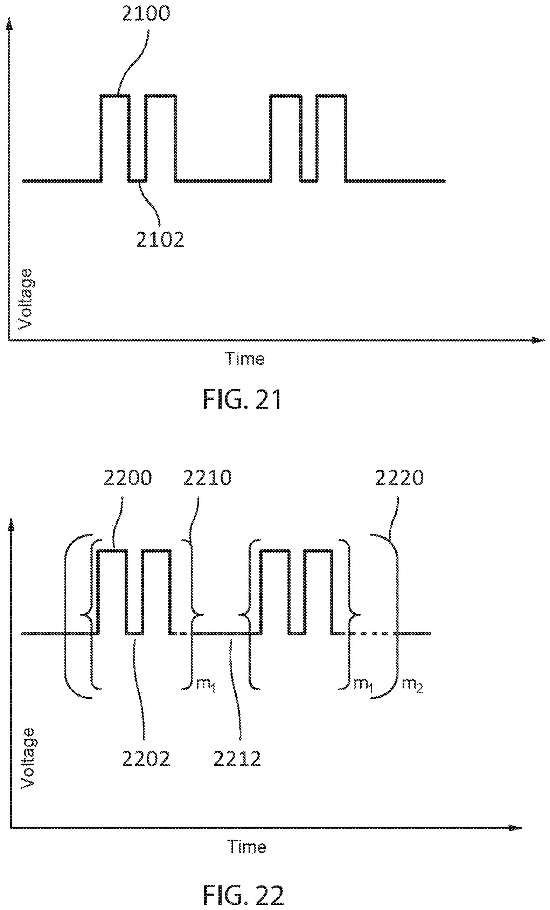

FIG. 21 illustrates a pulsed voltage waveform in the form of a sequence of rectangular double pulses, with each pulse, such as the pulse (2100) being associated with a pulse width or duration. The pulse width/duration can be about 0.5 microseconds, about 1 microsecond, about 5 microseconds, about 10 microseconds, about 25 microseconds, about 50 microseconds, about 100 microseconds, about 125 microseconds, about 140 microseconds, about 150 microseconds, including all values and sub-ranges in between. The pulsed waveform of FIG. 21 illustrates a set of monophasic pulses where the polarities of all the pulses are the same (all positive in FIG. 21, as measured from a zero baseline). In some embodiments, such as for irreversible electroporation applications, the height of each pulse (2100) or the voltage amplitude of the pulse (2100) can be in the range from about 400 volts, about 1,000 volts, about 5,000 volts, about 10,000 volts, about 15,000 volts, including all values and sub ranges in between. As illustrated in FIG. 21, the pulse (2100) is separated from a neighboring pulse by a time interval (2102), also sometimes referred to as a first time interval. The first time interval can be about 10 microseconds, about 50 microseconds, about 100 microseconds, about 200 microseconds, about 500 microseconds, about 800 microseconds, about 1 millisecond including all values and sub ranges in between, in order to generate irreversible electroporation.

FIG. 22 introduces a pulse waveform with the structure of a hierarchy of nested pulses. FIG. 22 shows a series of monophasic pulses such as pulse (2200) with pulse width/pulse time duration *w,* separated by a time interval (also sometimes referred to as a first time interval) such as (2202) of duration *t*₁ between successive pulses, a number *m*₁ of which are arranged to form a group of pulses (2210) (also sometimes referred to as a first set of pulses). Furthermore, the waveform has a number *m*₂ of such groups of pulses (also sometimes referred to as a second set of pulses) separated by a time interval (2212) (also sometimes referred to as a second time interval) of duration *f₂* between successive groups. The collection of *m*₂ such pulse groups, marked by (2220) in FIG. 22, constitutes the next level of the hierarchy, which can be referred to as a packet and/or as a third set of pulses. The pulse width and the time interval *t*₁ between pulses can both be in the range of microseconds to hundreds of microseconds, including all values and sub ranges in between. In some embodiments, the time interval *t*₂ can be at least three times larger than the time interval *t*₁. In some embodiments, the ratio *t*₂/*t*₁ can be in the range between about 3 and about 300, including all values and sub-ranges in between.

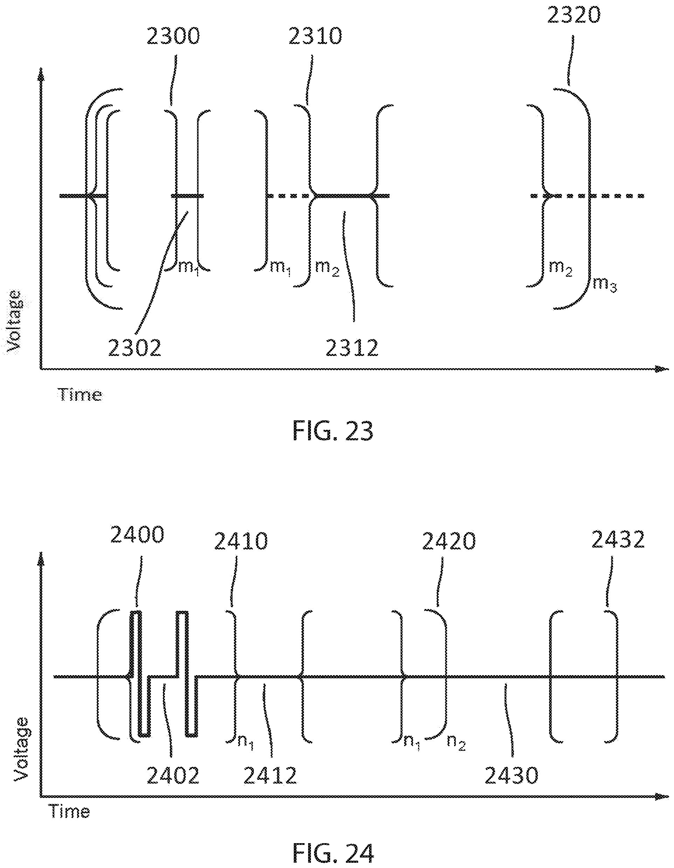

FIG. 23 further elaborates the structure of a nested pulse hierarchy waveform. In this figure, a series of *m*₁ pulses (individual pulses not shown) form a group of pulses (2300) (e.g., a first set of pulses). A series of *m*₂ such groups separated by an inter-group time interval (2310) of duration *t*₂ (e.g., a second time interval) between one group and the next form a packet 132 (e.g., a second set of pulses). A series of *m*₃ such packets separated by time intervals (2312) of duration *t*₃ (e.g., a third time interval) between one packet and the next form the next level in the hierarchy, a super-packet labeled (2320) (e.g., a third set of pulses) in the figure. In some embodiments, the time interval *t*₃ can be at least about thirty times larger than the time interval *t*₂. In some embodiments, the time interval *t*₃ can be at least fifty times larger than the time interval *t*₂. In some embodiments, the ratio t₃/t₂ can be in the range between about 30 and about 800, including all values and sub-ranges in between. The amplitude of the individual voltage pulses in the pulse hierarchy can be anywhere in the range from 500 volts to 7,000 volts or higher, including all values and sub ranges in between.

FIG. 24 provides an example of a biphasic waveform sequence with a hierarchical structure. In the example shown in the figure, biphasic pulses such as (2400) have a positive voltage portion as well as a negative voltage portion to complete one cycle of the pulse. There is a time delay (2402) (e.g., a first time interval) between adjacent cycles of duration *t*₁, and *n*₁ such cycles form a group of pulses (2410) (e.g., a first set of pulses). A series of *n*₂ such groups separated by an inter-group time interval (2412) (e.g., a second time interval) of duration *t*₂ between one group and the next form a packet (2420) (e.g., a second set of pulses). The figure also shows a second packet (2430), with a time delay (2432) (e.g., a third time interval) of duration *t*₃ between the packets. Just as for monophasic pulses, higher levels of the hierarchical structure can be formed as well. The amplitude of each pulse or the voltage amplitude of the biphasic pulse can be anywhere in the range from 500 volts to 7,000 volts or higher, including all values and sub ranges in between. The pulse width/pulse time duration can be in the range from nanoseconds or even sub-nanoseconds to tens of microseconds, while the delays *t*₁ can be in the range from zero to several microseconds. The inter-group time interval *t*₂ can be at least ten times larger than the pulse width. In some embodiments, the time interval *t*₃ can be at least about twenty times larger than the time interval *t*₂. In some embodiments, the time interval *t*₃ can be at least fifty times larger than the time interval *t*₂.

Embodiments disclosed herein include waveforms structured as hierarchical waveforms that include waveform elements/pulses at various levels of the hierarchy. The individual pulses such as (2200) in FIG. 22 comprise the first level of the hierarchy, and have an associated pulse time duration and a first time interval between successive pulses. A set of pulses, or elements of the first level structure, form a second level of the hierarchy such as the group of pulses/second set of pulses (2210) in FIG. 22. Among other parameters, associated with the waveform are parameters such as a total time duration of the second set of pulses (not shown), a total number of first level elements/first set of pulses, and second time intervals between successive first level elements that describe the second level structure/second set of pulses. In some embodiments, the total time duration of the second set of pulses can be between about 20 microseconds and about 10 milliseconds, including all values and subranges in between. A set of groups, second set of pulses, or elements of the second level structure, form a third level of the hierarchy such as the packet of groups/third set of pulses (2220) in FIG. 22. Among other parameters, there is a total time duration of the third set of pulses (not shown), a total number of second level elements/second set of pulses, and third time intervals between successive second level elements that describe the third level structure/third set of pulses. In some embodiments, the total time duration of the third set of pulses can be between about 60 microseconds and about 200 milliseconds, including all values and sub ranges in between. The generally iterative or nested structure of the waveforms can continue to a higher plurality of levels, such as ten levels of structure, or more.

In some embodiments, hierarchical waveforms with a nested structure and hierarchy of time intervals as described herein are useful for irreversible electroporation ablation energy delivery, providing a good degree of control and selectivity for applications in different tissue types. A variety of hierarchical waveforms can be generated with a suitable pulse generator. It is understood that while the examples herein identify separate monophasic and biphasic waveforms for clarity, it should be noted that combination waveforms, where some portions of the waveform hierarchy are monophasic while other portions are biphasic, can also be generated/implemented.

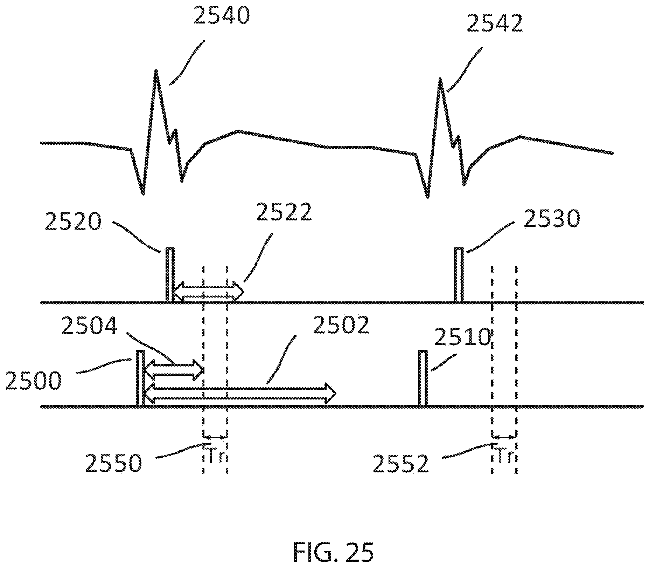

In some embodiments, the ablation pulse waveforms described herein are applied during the refractory period of the cardiac cycle so as to avoid disruption of the sinus rhythm of the heart. In some embodiments, a method of treatment includes electrically pacing the heart with a cardiac stimulator to ensure pacing capture to establish periodicity and predictability of the cardiac cycle, and then defining a time window within the refractory period of the cardiac cycle within which one or more pulsed ablation waveforms can be delivered. FIG. 25 illustrates an example where both atrial and ventricular pacing is applied (for instance, with pacing leads or catheters situated in the right atrium and right ventricle respectively). With time represented on the horizontal axis, FIG. 25 illustrates a series of ventricular pacing signals such as (2500) and (2510), and a series of atrial pacing signals (2520, 2530), along with a series of ECG waveforms (2540, 2542) that are driven by the pacing signals. As indicated in FIG. 25 by the thick arrows, there is an atrial refractory time window (2522) and a ventricular refractory time window (2502) that respectively follow the atrial pacing signal (2522) and the ventricular pacing signal (2500). As shown in FIG. 25, a common refractory time window (2550) of duration *Tᵣ* can be defined that lies within both atrial and ventricular refractory time windows (2522, 2502). In some embodiments, the electroporation ablation waveform(s) can be applied in this common refractory time window (2550). The start of this refractory time window (2522) is offset from the pacing signal (2500) by a time offset (2504) as indicated in FIG. 25. The time offset (2504) can be smaller than about 25 milliseconds, in some embodiments. At the next heartbeat, a similarly defined common refractory time window (2552) is the next time window available for application of the ablation waveform(s). In this manner, the ablation waveform(s) may be applied over a series of heartbeats, at each heartbeat remaining within the common refractory time window. In one embodiment, each packet of pulses as defined above in the pulse waveform hierarchy can be applied over a heartbeat, so that a series of packets is applied over a series of heartbeats, for a given electrode set.

As used herein, the terms "about" and/or "approximately" when used in conjunction with numerical values and/or ranges generally refer to those numerical values and/or ranges near to a recited numerical value and/or range. In some instances, the terms "about" and "approximately" may mean within ± 10% of the recited value. For example, in some instances, "about 100 [units]" may mean within ± 10% of 100 (e.g., from 90 to 110). The terms "about" and "approximately" may be used interchangeably.

Some embodiments described herein relate to a computer storage product with a non-transitory computer-readable medium (also may be referred to as a non-transitory processor-readable medium) having instructions or computer code thereon for performing various computer-implemented operations. The computer-readable medium (or processor-readable medium) is non-transitory in the sense that it does not include transitory propagating signals per se (e.g., a propagating electromagnetic wave carrying information on a transmission medium such as space or a cable). The media and computer code (also may be referred to as code or algorithm) may be those designed and constructed for the specific purpose or purposes. Examples of non-transitory computer-readable media include, but are not limited to, magnetic storage media such as hard disks, floppy disks, and magnetic tape; optical storage media such as Compact Disc/Digital Video Discs (CD/DVDs), Compact Disc-Read Only Memories (CD-ROMs), and holographic devices; magneto-optical storage media such as optical disks; carrier wave signal processing modules; and hardware devices that are specially configured to store and execute program code, such as Application-Specific Integrated Circuits (ASICs), Programmable Logic Devices (PLDs), Read-Only Memory (ROM) and Random-Access Memory (RAM) devices. Other embodiments described herein relate to a computer program product, which may include, for example, the instructions and/or computer code disclosed herein.

The systems, devices, and/or methods described herein may be performed by software (executed on hardware), hardware, or a combination thereof. Hardware modules may include, for example, a general-purpose processor (or microprocessor or microcontroller), a field programmable gate array (FPGA), and/or an application specific integrated circuit (ASIC). Software modules (executed on hardware) may be expressed in a variety of software languages (e.g., computer code), including C, C++, Java®, Ruby, Visual Basic®, and/or other object-oriented, procedural, or other programming language and development tools. Examples of computer code include, but are not limited to, micro-code or micro-instructions, machine instructions, such as produced by a compiler, code used to produce a web service, and files containing higher-level instructions that are executed by a computer using an interpreter. Additional examples of computer code include, but are not limited to, control signals, encrypted code, and compressed code.

The specific examples and descriptions herein are exemplary in nature and embodiments may be developed by those skilled in the art based on the material taught herein without departing from the scope of the present invention, which is limited only by the attached claims.

## Claims

1. A system, comprising:
a signal generator (122) configured for generating a pulse waveform; and
an ablation device (900) coupled to the signal generator the ablation device including:
a catheter shaft (910);
a set of spines (914) coupled to the distal end of the catheter shaft; and
a set of independently addressable electrodes (916, 918) formed on a surface of each of the spines,
each electrode of the set of electrodes having an insulated electrical lead associated therewith, the insulated electrical leads disposed in a body of each of the set of spines,
the insulated electrical leads having an insulation configured to sustain an electrical potential difference of at least about 700 V across its thickness without dielectric breakdown such that the set of electrodes can receive the pulse waveform and deliver electrical energy to ablate tissue through irreversible electroporation,
the set of spines deployable to form an asymmetric expanded structure in which a subset of the set of electrodes lies in a distal end plane that is orthogonal to a longitudinal axis of the catheter shaft and passes through a distal boundary of the expanded structure.

2. The system of claim 1, further comprising a cardiac stimulator (128) configured for generating a pacing signal for cardiac stimulation during use, the cardiac stimulator communicably coupled to the signal generator and further configured for transmitting an indication of the pacing signal to the signal generator,
the signal generator further configured for generating the pulse waveform in synchronization with the indication of the pacing signal.

3. The system of claim 2, the signal generator further configured for generating the pulse waveform with a time offset with respect to the indication of the pacing signal.

4. The system of claim 1, each electrode of the set of electrodes having a surface area from about 0.5 mm² to about 20 mm².

5. The system of claim 1, each spine of the set of spines having a cross-sectional area from about 0.2 mm² to about 15 mm².

6. The system of claim 1, the set of spines forming a delivery assembly at a distal portion of the ablation device, the delivery assembly configured for transforming between a first configuration where the set of spines bow radially outward from a longitudinal axis of the ablation device, and a second configuration where the set of spines are arranged generally parallel to the longitudinal axis of the ablation device.

7. The system of claim 1, the ablation device further configured for delivering the pulse waveform to the tissue during use via the set of electrodes by configuring a first electrode of the set of electrodes as an anode and a second electrode of the set of electrodes as a cathode.

8. The system of claim 1, wherein a cross-sectional area at a first plane of the expanded structure is different than a cross-sectional area at a second plane of the expanded structure, the first plane and the second plane being orthogonal to a longitudinal axis of the catheter shaft.

9. The system of claim 1, wherein the set of spines converge at their distal ends at a point inside the expanded structure.

10. The system of claim 1, wherein the set of electrodes formed on the surface of each of the one or more spines includes a distal electrode and a proximal electrode, the distal electrode of each set of electrodes being configured as an anode and the proximal electrode of each set of electrodes being configured as a cathode.

11. The system of claim 10, wherein, when the set of spines form the expanded structure, at least one distal electrode is configured to engage an atrial wall to generate lesions by activation of the at least one distal electrode.

12. The system of claim 1, wherein the set of spines are configured to form an expanded structure having a distal portion being more bulbous than a proximal portion.

13. The system of claim 1, the ablation device further configured for delivering the pulse waveform to the tissue during use via the set of electrodes by configuring a first set of electrodes as an anode and a second set of electrodes as a cathode.

## Patentansprüche

1. System, das Folgendes umfasst:
einen Signalgenerator (122), der zur Erzeugung einer Pulswellenform konfiguriert ist; und
eine Ablationsvorrichtung (900), die mit dem Signalgenerator verbunden ist, wobei die
Ablationsvorrichtung Folgendes einschließt:
einen Katheterschaft (910);
einen Satz von Speichen (914), die mit dem distalen Ende des Katheterschafts verbunden sind; und
einen Satz von unabhängig adressierbaren Elektroden (916, 918), die auf einer Oberfläche von jeder der Speichen ausgebildet sind,
wobei jede Elektrode des Satzes von Elektroden eine isolierte elektrische Leitung aufweist, die damit in Verbindung steht, die isolierten elektrischen Leitungen in einem Körper von jeder des Satzes von Speichen angeordnet ist, die isolierten elektrischen Leitungen eine Isolierung aufweisen, die zur Erhaltung einer elektrischen Potenzialdifferenz von mindestens etwa 700 V über ihre Dicke ohne dielektrischen Durchschlag konfiguriert ist, sodass der Satz von Elektroden die Pulswellenform empfangen und elektrische Energie abgeben kann, um Gewebe durch irreversible Elektroporation zu ablatieren,
wobei der Satz von Speichen, die zur Bildung einer asymmetrischen expandierten Struktur einsetzbar sind, in der ein Teilsatz des Satzes von Elektrode in einer Ebene des distalen Endes liegt, die rechtwinklig zu einer Längsachse des Katheterschafts verläuft und durch eine distale Abgrenzung der expandierten Struktur geht.

2. System nach Anspruch 1, das weiter einen Herzstimulator (128) umfasst, der zur Erzeugung eines Schrittmachersignals zur Herzstimulation während der Verwendung konfiguriert ist, wobei der Herzstimulator mit dem Signalgenerator kommunizierend verbunden ist und weiter zur Übertragung einer Anzeige des Schrittmachersignals an den Signalgenerator konfiguriert ist,
wobei der Signalgenerator weiter zur Erzeugung der Pulswellenform synchron mit der Anzeige des Schrittmachersignals konfiguriert ist.

3. System nach Anspruch 2, wobei der Signalgenerator weiter zur Erzeugung der Pulswellenform mit Zeitversatz in Bezug auf die Anzeige des Schrittmachersignals konfiguriert ist.

4. System nach Anspruch 1, wobei jede Elektrode des Satzes von Elektroden eine Oberfläche von etwa 0,5 mm² bis etwa 20 mm² aufweist.

5. System nach Anspruch 1, wobei jede Speiche des Satzes von Speichen eine Querschnittsfläche von etwa 0,2 mm² bis etwa 15 mm² aufweist.

6. System nach Anspruch 1, wobei der Satz von Speichen eine Abgabeanordnung an einem distalen Teil der Ablationsvorrichtung bildet, wobei die Abgabeanordnung zur Umwandlung zwischen einer ersten Konfiguration, wo der Satz von Speichen radial nach außen von einer Längsachse der Ablationsvorrichtung gebogen ist, und einer zweiten Konfiguration konfiguriert ist, wo der Satz von Speichen im Allgemeinen parallel zu der Längsachse der Ablationsvorrichtung angeordnet ist.

7. System nach Anspruch 1, wobei die Ablationsvorrichtung weiter zur Abgabe der Pulswellenform an das Gewebe während der Verwendung über den Satz von Elektroden durch Konfigurieren einer ersten Elektrode des Satzes von Elektroden als eine Anode und einer zweiten Elektrode des Satzes von Elektroden als eine Kathode konfiguriert ist.

8. System nach Anspruch 1, wobei eine Querschnittsfläche an einer ersten Ebene der expandierten Struktur anders ist als eine Querschnittsfläche an einer zweiten Ebene der expandierten Struktur, wobei die erste Ebene und die zweite Ebene rechtwinklig zu einer Längsachse des Katheterschafts verlaufen.

9. System nach Anspruch 1, wobei der Satz von Speichen an ihren distalen Enden an einem Punkt innerhalb der expandierten Struktur zusammenläuft.

10. System nach Anspruch 1, wobei der Satz von Elektroden, die auf der Oberfläche von jeder der einen oder mehreren Speichen ausgebildet sind, eine distale Elektrode und eine proximale Elektrode einschließt, wobei die distale Elektrode von jedem Satz von Elektroden als eine Anode konfiguriert ist und die proximale Elektrode von jedem Satz von Elektroden als eine Kathode konfiguriert ist.

11. System nach Anspruch 10, wobei, wenn der Satz von Speichen die expandierte Struktur bildet, mindestens eine distale Elektrode zum Eingreifen in eine atriale Wand konfiguriert ist, um Läsionen durch Aktivierung der mindestens einen distalen Elektrode zu erzeugen.

12. System nach Anspruch 1, wobei der Satz von Speichen konfiguriert ist, um eine expandierte Struktur zu bilden, die einen distalen Teil aufweist, der bauchiger ist als ein proximaler Teil.

13. System nach Anspruch 1, wobei die Ablationsvorrichtung weiter zur Abgabe der Pulswellenform an das Gewebe während der Verwendung über den Satz von Elektroden durch Konfigurieren eines ersten Satzes von Elektroden als eine Anode und eines zweiten Satzes von Elektroden als eine Kathode konfiguriert ist.

## Revendications

1. Système, comprenant :
un générateur de signal (122) configuré pour générer une forme d'onde impulsionnelle ; et
un dispositif d'ablation (900) couplé au générateur de signal, le dispositif d'ablation comportant :
une tige de cathéter (910) ;
un ensemble de pointes (914) couplées à l'extrémité distale de la tige de cathéter ; et
un ensemble d'électrodes adressables de manière indépendante (916, 918) formées sur une surface de chacune des pointes,
chaque électrode de l'ensemble d'électrodes ayant un fil électrique isolé qui lui est associé, les fils électriques isolés étant disposés dans un corps de chaque pointe de l'ensemble de pointes, les fils électriques isolés ayant une isolation configurée pour supporter une différence de potentiel électrique d'au moins 700 V environ sur son épaisseur sans claquage diélectrique de telle façon que l'ensemble d'électrodes peut recevoir la forme d'onde impulsionnelle et fournir de l'énergie électrique pour l'ablation de tissu par une électroporation irréversible,
l'ensemble de pointes pouvant être déployé pour former une structure expansée asymétrique dans laquelle un sous-ensemble de l'ensemble d'électrodes se situe dans un plan d'extrémité distale qui est orthogonal à un axe longitudinal de la tige de cathéter et traverse une limite distale de la structure expansée.

2. Système selon la revendication 1, comprenant en outre un stimulateur cardiaque (128) configuré pour générer un signal stimulateur pour la stimulation cardiaque à l'utilisation, le stimulateur cardiaque étant couplé de manière communicante au générateur de signal et en outre configuré pour transmettre une indication du signal stimulateur au générateur de signal,
le générateur de signal étant en outre configuré pour générer la forme d'onde impulsionnelle en synchronisation avec l'indication du signal stimulateur.

3. Système selon la revendication 2, le générateur de signal étant en outre configuré pour générer la forme d'onde impulsionnelle avec un décalage temporel par rapport à l'indication du signal stimulateur.

4. Système selon la revendication 1, chaque électrode de l'ensemble d'électrodes ayant une aire de surface d'environ 0,5 mm² à environ 20 mm².

5. Système selon la revendication 1, chaque pointe de l'ensemble de pointes ayant une aire de section transversale d'environ 0,2 mm² à environ 15 mm².

6. Système selon la revendication 1, l'ensemble de pointes formant un ensemble de délivrance au niveau d'une partie distale du dispositif d'ablation, l'ensemble de délivrance étant configuré pour se transformer entre une première configuration dans laquelle les pointes de l'ensemble de pointes sont arquées radialement vers l'extérieur à partir d'un axe longitudinal du dispositif d'ablation, et une deuxième configuration dans laquelle les pointes de l'ensemble de pointes sont disposées de manière généralement parallèle à l'axe longitudinal du dispositif d'ablation.

7. Système selon la revendication 1, le dispositif d'ablation étant en outre configuré pour délivrer la forme d'onde impulsionnelle au tissu, à l'utilisation, par le biais de l'ensemble d'électrodes en configurant une première électrode de l'ensemble d'électrodes en tant qu'anode et une deuxième électrode de l'ensemble d'électrodes en tant que cathode.

8. Système selon la revendication 1, dans lequel une aire de section transversale au niveau d'un premier plan de la structure expansée est différente d'une aire de section transversale au niveau d'un deuxième plan de la structure expansée, le premier plan et le deuxième plan étant orthogonaux à un axe longitudinal de la tige de cathéter.

9. Système selon la revendication 1, dans lequel les pointes de l'ensemble de pointes convergent au niveau de leurs extrémités distales en un point à l'intérieur de la structure expansée.

10. Système selon la revendication 1, dans lequel l'ensemble d'électrodes formées sur la surface de chacune desdites une ou plusieurs pointes comporte une électrode distale et une électrode proximale, l'électrode distale de chaque ensemble d'électrodes étant configurée en tant qu'anode et l'électrode proximale de chaque ensemble d'électrodes étant configurée en tant que cathode.

11. Système selon la revendication 10, dans lequel, lorsque l'ensemble de pointes forme la structure expansée, au moins une électrode distale est configurée pour venir en prise avec une paroi atriale afin de générer des lésions par l'activation de ladite au moins une électrode distale.

12. Système selon la revendication 1, dans lequel l'ensemble de pointes est configuré pour former une structure expansée ayant une partie distale qui est plus renflée qu'une partie proximale.

13. Système selon la revendication 1, le dispositif d'ablation étant en outre configuré pour délivrer la forme d'onde impulsionnelle au tissu, à l'utilisation, par le biais de l'ensemble d'électrodes en configurant un premier ensemble d'électrodes en tant qu'anode et un deuxième ensemble d'électrodes en tant que cathode.
